# EUROPEAN PATENT SPECIFICATION

(11) **EP 4 265 740 B1**
(45) Date of publication and mention of the grant of the patent: **08.04.2026**
(21) Application number: 23177369.8
(22) Date of filing: 28.02.2014
(51) Int. Cl.: C12Q 1/6886

(54) **METHODS FOR PREDICTING RISK OF METASTASIS IN CUTANEOUS MELANOMA**
VERFAHREN ZUR VORHERSAGE DES RISIKOS VON METASTASEN BEI HAUTMELANOMEN
MÉTHODES DE PRÉDICTION DU RISQUE DE MÉTASTASE DANS UN MÉLANOME CUTANÉ

(30) Priority: 14.03.2013 US 201361783755 P
(43) Date of publication of application: 25.10.2023
(62) Divisional of application: 18187484.3
(73) Proprietor: Castle Biosciences, Inc., Friendswood, TX 77546 (US)
(72) Inventor: COOK, Robert Willis, Friendswood, 77546 (US); MAETZOLD, Derek, Friendswood, 77546 (US); OESCHLAGER, Kristen, Friendswood, 77546 (US)
(74) Representative: Zwicker, Jörk

(56) References cited:
- WO-A2-2006/092610
- WO-A2-2009/132126
- WO-A2-2011/039734
- ANDREAS MAUERER ET AL: "Identification of new genes associated with melanoma", EXPERIMENTAL DERMATOLOGY, vol. 20, no. 6, 16 March 2011 (2011-03-16), pages 502 - 507, XP055122228, ISSN: 0906-6705, DOI: 10.1111/j.1600-0625.2011.01254.x
- MICHAEL D. ONKEN ET AL: "An Accurate, Clinically Feasible Multi-Gene Expression Assay for Predicting Metastasis in Uveal Melanoma", THE JOURNAL OF MOLECULAR DIAGNOSTICS, vol. 12, no. 4, 1 July 2010 (2010-07-01), pages 461 - 468, XP055523610, ISSN: 1525-1578, DOI: 10.2353/jmoldx.2010.090220

## Description

### BACKGROUND OF THE INVENTION

Cutaneous melanoma (CM) is an aggressive form of cancer presenting over 76,000 diagnosed cases in 2012.¹ CM tumors develop through a number of discreet stages during the progression from a benign melanocytic nevus to a malignant metastatic tumor. Generally, benign nevi present as thin, pigmented lesions. After the acquisition of key genetic mutations and the initiation of cytoarchitectural modifications leading to shallow invasion of the skin, the lesions begin growing radially, a process referred to as the radial growth phase. Upon escape from growth control mediated by surrounding keratinocytes, stromal invasion to deeper regions of the dermis occurs, marking the progression to the vertical growth phase. The vertical growth phase, along with the genetic alterations that accompany this process, is thought to be the critical stepping stone in the development of metastatic melanoma.

As is common in many other cancers, if CM is detected in the early stages of tumor progression and appropriately treated, then a long-term metastasis free and high overall survival following diagnosis is likely for the majority of patients.^{2,3} For example, subjects diagnosed with low risk, stage I CM tumors have a 5-year overall survival rate of 91-97%.³ A number of histological factors are used to stage CM and are associated with prognosis. These factors include Breslow thickness, mitotic index, ulceration, and spread of disease from the primary tumor to sentinel and/or regional lymph nodes.³⁻⁷ Tumor stage is determined based upon these histopathological parameters using the well-known TNM (T = primary tumor, N = regional lymph nodes, M = distant metastases) system that defines stages 0-IV.³ The TNM staging system is highly accurate for metastasis-free survival for stage 0, *in situ,* melanomas that do not invade the dermal layer (5-year survival of 99%), and stage IV melanomas (5-year survival <10%), in which distant metastasis was detected at the time of primary diagnosis. Metastasis and short-term survival has been documented for subjects with stage I disease, with 5-10% of stage I tumors reporting metastatic activity despite successful surgical intervention (wide local excision with adequate clear margins). So, while the majority of patients with clinical stage I disease have a low chance of metastasis risk disease some patients will develop metastatic disease.

Prognosis for clinical stage II and stage III cases has poor accuracy as there is a large range within each stage and a larger overlap between the stages in 5-year survival rates. Under the current staging system, the 5-year survival rate for clinical stage II subjects is 53-82%, while the stage III 5-year survival is rate 22-68%.^{3,8} The distinguishing hallmark between clinical stage II and stage III tumors, and stage I and stage III tumors, is the presence of localized metastasis of CM cells in the sentinel lymph node (SLN) following a SLN biopsy procedure. Patients with a positive SLN are clinically upstaged to stage III. However, high false negative rates and disease recurrence are associated with histological analysis of SLNs as evidenced by the wide ranges of metastatic free survival and overall survival in stage II and III patients. Immunohistochemical and genetic amplification techniques designed to improve the common hematoxylin and eosin staining methods for detection of regional disease have been developed, but only provide marginal improvements^{9,10}. Biomarkers have been identified in SLN tissue, and analyzed to improve the ability to recognize CM cells in SLNs, however, these methods have shown limited improvement in accuracy and are compounded by extensive tissue sampling from invasive biopsy of the lymph node and have not been adopted for clinical use.^{6,11} In addition, melanomas can enter the blood directly by intravasating into venous capillaries. Thus, the low sensitivity of SLN biopsy may relate to a direct hematogenous metastatic event versus an inaccurate SLN biopsy result. Regardless of the clinical deficiencies observed for the SLNB procedure, it remains the single best prognostic factor for predicting metastatic risk for CM patients, and is included in multiple association guidelines for clinical management of CM disease.

Inaccurate prognosis for metastatic risk has profound effects upon patients that are treated according to a population approach rather than an individual or personalized approach. For example, CM patients categorized as stage III through the use of current histological techniques, but who have an actual individual risk of metastasis that is low (false positive), are inappropriately exposed to over-treatment that includes enhanced surveillance, nodal surgery, and chemotherapy.¹² Similarly, who are SLN negative, that is no CM cells were found within the SLN's, remain stage I or II disease but who actually have a high risk for metastasis (false negative), are at risk of under-treatment. In addition, SLN biopsy is an intervention that typically occurs under general anesthesia, exposes patients to significant clinical complications, and has a low positivity rate. For example, National Comprehensive Cancer Network (NCCN) Guidelines^{®} currently recommend that patients with CM staged at 1b (Breslow's thickness ≥ 0.75mm but <1.00 mm or presence of ≥ 1 mitosis at any Breslow's thickness) are recommended to undergo SLN biopsy yet only 5% of SLN yield positivity. Meaning that of 20 patients with stage 1b melanomas who undergo SLN biopsy, 19 will be negative and exposed to a surgical complication of SLN biopsy.¹² Similarly, all pathologic stage II patients (Breslow's thickness >1.0mm) are recommended to undergo SLN biopsy yet only 18% will have a positive SLN.^{11,13} In addition, a positive SLN biopsy results in recommendations for a complete regional lymph dissection which exposes patients to significant clinical complications, such as lymphedema, and has a low positivity rate.

To this end, gene expression profile (GEP) signatures have been developed and some have been shown to have powerful prognostic capabilities in a number of malignant diseases¹⁴⁻¹⁸. One such signature has been used for prognostication of uveal melanoma, a tumor of melanocytic origin that develops in the eye. Like cutaneous melanoma, treatment of the primary uveal tumor is highly effective. Two to four percent of uveal melanoma patients present with evidence of clinical metastasis at the time of diagnosis, yet up to 50% of uveal melanoma patients develop systemic metastases within five years of diagnosis regardless of primary eye tumor treatment (radiation therapy or enucleation)¹⁹. This means that a micrometastatic event has occurred in approximately 50% of uveal melanoma patients prior to primary eye tumor treatment. A GEP signature has recently been developed that can accurately distinguish uveal melanoma tumors that have a low risk of metastasis from those that have a high risk^{14,20}. To assess genetic expression real-time polymerase chain reaction (RT-PCR) analysis is performed for fifteen genes (twelve discriminating genes and three control genes) that are differentially expressed in tumors with known metastatic activity compared to tumors with no evidence of metastasis. The uveal melanoma gene signature separates cases into a low risk group that has greater than 95% metastasis free survival five years after diagnosis, and a high risk group with less than 20% metastasis free survival at the same time point. The signature has been extensively validated in the clinical setting, and has been shown to provide a significant improvement in prognostic accuracy compared to classification by TNM staging criteria^{20,21}

A number of groups have published genomic analysis of tumors in cutaneous melanoma²²⁻²⁹. While some studies have focused on the genetic alterations in malignant melanoma cells compared to normal melanocytes, others have compared benign nevi to tumors in the radial or vertical growth phases, or primary tumor to metastatic tumors. At the time the studies contained within this patent were designed and implemented (2010), no evidence could be found in the literature or other public domain sources that indicated a gene expression profile test focused solely on the primary melanoma tumor could be developed for the clinical application of predicting metastasis in patients with CM.^{22-24,27-29} In addition, all studies utilized fresh frozen CM samples rather than formalin fixed paraffin embedded (FFPE) tumor tissue. All of the studies related to this invention have only used FFPE primary tumor tissue.

### SUMMARY OF THE INVENTION

There is a need in the art for a more accurate and objective method of predicting which tumors display aggressive metastatic activity. Development of an accurate molecular footprint, such as the gene expression profile assay encompassed by the invention disclosed herein, by which CM metastatic risk could be assessed from primary tumor tissue would be a significant advance forward for the field. Inaccurate prognosis for metastatic risk has profound effects upon patients including inappropriate exposure to over-treatment that includes enhanced surveillance, nodal surgery, and chemotherapy. Patients with inaccurate diagnoses are also at risk of under-treatment; that is, cancer cells are not seen in the sentinel lymph node although they are present and may have already spread to other regional lymph nodes or other parts of the body. A false-negative biopsy result gives the patient and the doctor a false sense of security about the extent of cancer in the patient's body. In addition, SLN biopsy exposes patients to significant clinical complications, such as lymphedema, and has a low positivity rate.

The invention as disclosed herein provides a method for predicting risk of metastasis, overall survival, or both, in a patient with a primary cutaneous melanoma tumor, the method comprising: (a) measuring gene expression levels of at least eight genes selected from the group consisting of BAP1_varA, BAP1_varB, MGP, SPP1, CXCL14, CLCA2, S100A8, BTG1, SAP130, ARG1, KRT6B, GJA, ID2, EIF1B, S100A9, CRABP2, KRT14, ROBO1, RBM23, TACSTD2, DSC1, SPRR1B, TRIM29, AQP3, TYRP1, PPL, LTA4H, and CST6, in a sample of the primary cutaneous melanoma tumor, wherein the at least eight genes are SPP1, MGP, KRT6B, PPL, RBM23, AQP3, CXCL14 and GJA1, measuring gene expression levels of the at least eight genes comprises measurement of a level of fluorescence by a sequence detection system following RT-PCR of the at least eight genes; (b) determining a patient gene-expression profile signature comprising the gene expression levels of the at least eight genes; (c) comparing the patient gene-expression profile signature to a gene-expression profile of a predictive training set; and (d) providing an indication as to a risk of metastasis, overall survival or both for the primary cutaneous melanoma tumor when the patient gene expression profile indicates that the expression levels of at the least eight genes are altered in a predictive manner as compared to the gene expression profile of the predictive training set.

This disclosure provides a more objective method that more accurately predicts which melanoma tumors display aggressive metastatic activity and result in decreased patient overall survival. Development of an accurate molecular footprint, such as the gene expression profile assay encompassed by the invention disclosed herein, by which CM metastatic risk and patient overall survival could be assessed from primary tumor tissue would be a significant advance forward for the field leading to decreased loss of life, less patient suffering, more efficient treatments and use of resources.

Further embodiments of the invention are disclosed in the accompanying claims. Specific embodiments of the invention will become evident from the following more detailed description of certain embodiments and the claims.

### BRIEF DESCRIPTION OF THE DRAWINGS

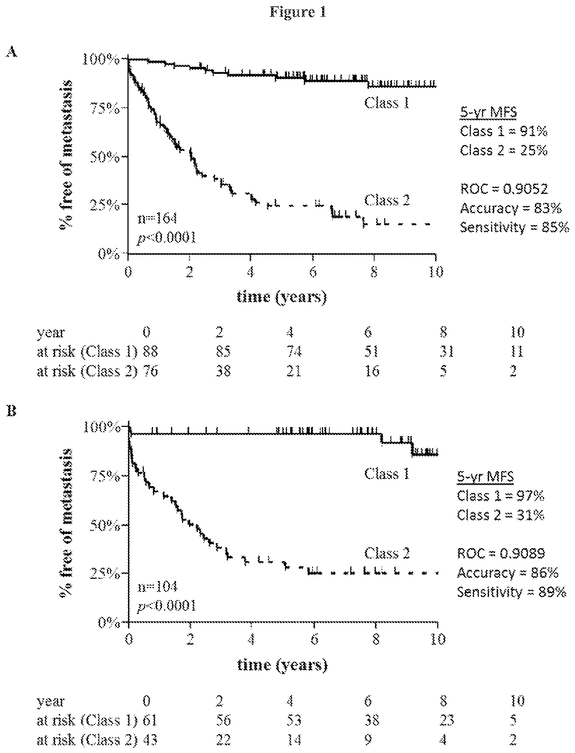

**Figure** 1 depicts statistical analysis and graphs illustrating Kaplan-Meier (K-M) analysis of metastasis free survival (MFS) for CM cases predicted to be at low-risk (class 1) or high-risk (class 2) of metastasis according to a radial basis machine (RBM) modeling algorithm. 5-yr MFS for the 164 sample training set (**A**) is 91% for class 1 cases and 25% for class 2 cases (p<0.0001). 5-yr MFS for the independent 104 sample validation set (**B**) is 97% for class 1 compared to 31% for class 2 (*p*<0.0001)*.* Accuracy of the training set model, as measured by ROC, is 0.9052, compared to 0.9089 for the validation cohort, each reflecting clinically valuable models.

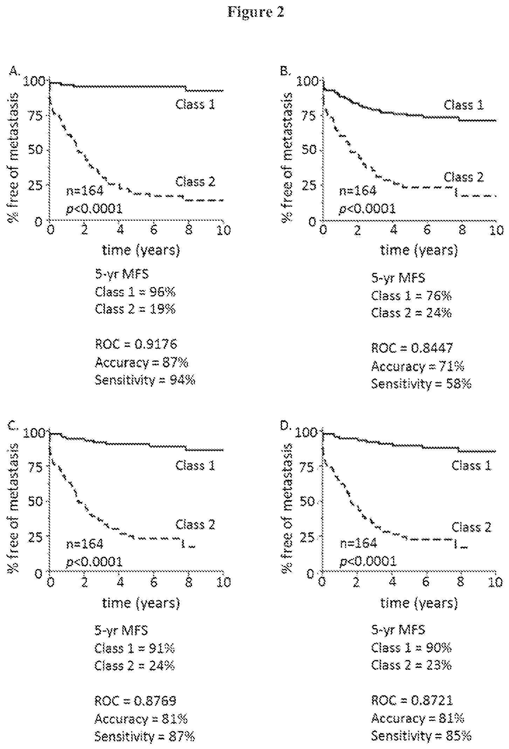

**Figure** 2 depicts statistical and K-M analysis of the 164 sample training set when metastasis is predicted using partition tree **(A),** K-nearest neighbor **(B),** logistic regression **(C),** or discriminant analysis **(D)** modeling algorithms. Highly significant differences in K-M MFS curves for class 1 and class 2 cohorts are observed with each modeling method. Accuracy is highest when analysis is performed with the partition tree model (ROC=0.9176; accuracy=87%; sensitivity=94%), while the other models are also statistically accurate, with ROC > 0.8.

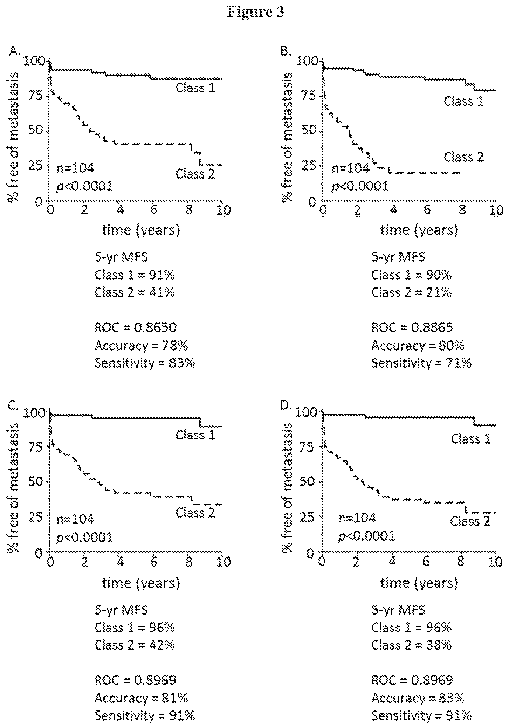

**Figure 3** depicts the same statistics and graphs as Figure 2, but with partition tree **(A),** K-nearest neighbor **(B),** logistic regression **(C),** or discriminant analysis **(D)** used to analyze the independent 104 sample validation set. Significant differences between class 1 and class 2 K-M curves are observed using each modeling method. Algorithm accuracy is highest for the logistic regression and discriminant analysis models (ROC=0.8969), and the same was true for sensitivity, or accuracy of predicting class 2 high risk cases (sensitivity=91%).

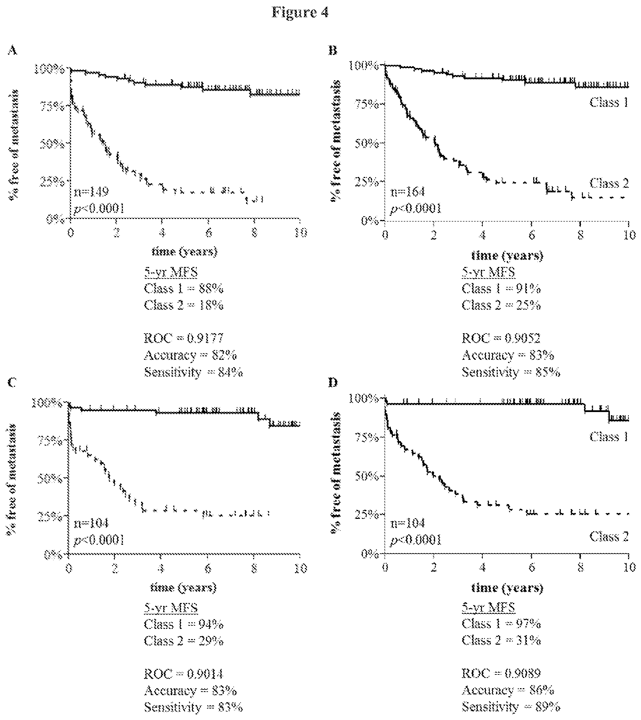

**Figure 4** depicts statistics and K-M analysis reflecting the impact of stage 0 *in situ* melanomas on training set predictive power. Removing stage 0 cases from the original training and validation cohorts produces a 149 sample training set (A) and independent 104 sample validation set **(C).** Significant differences are observed in class 1 and class 2 K-M curves for both cohorts (*p*<0.0001), and metastatic risk prediction is accurate in both the training and validation sets (ROC=0.9177 and 0.9014, respectively). Analysis was also performed following inclusion of all stage 0 samples in the training set, producing a training cohort of 164 samples **(B)** that exhibited comparable accuracy (ROC=0.9052; Accuracy=83%; sensitivity=85%) compared to the 149 sample training set. No dramatic differences are observed when the independent 104 sample validation set is trained using the 164 sample cohort **(D)** compared to **(C).**

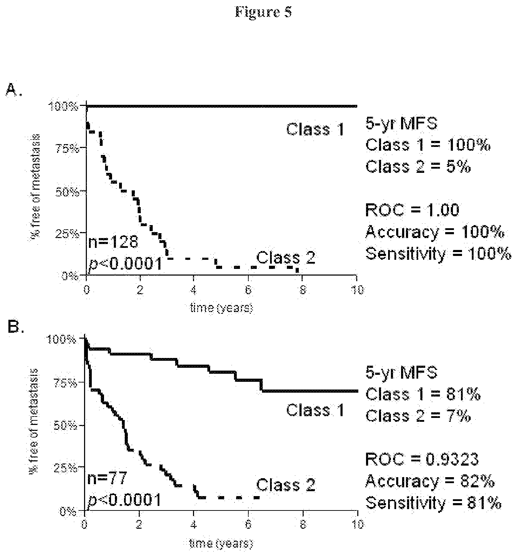

**Figure 5** depicts MFS statistics and accuracy of the genetic signature when used to predict metastatic risk in superficial spreading **(A)** or nodular **(B)** type CM tumors. K-M MFS was 100% for superficial spreading cases predicted to be class 1, and only 5% for cases predicted to be class 2 (p<0.0001). The predictive model had an accuracy of 100% for predicting both classes, and ROC=1.00. Prediction of risk for nodular tumors was less accurate, with an ROC = 0.9323, accuracy of 82%, and sensitivity of 81%, but K-M analysis reflects a significant difference between the 81% 5-year MFS for Class 1 and the 7% MFS for Class 2 cases (p<0.0001).

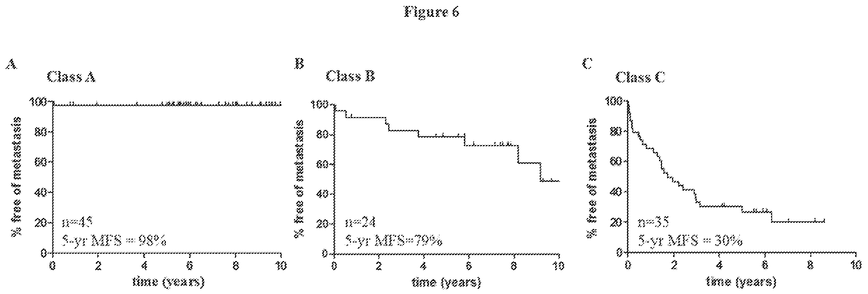

**Figure 6** depicts MFS graphs and statistical analysis following a tri-modal prediction for CM cases at low-risk (class A), intermediate risk (class B) or high-risk (class C) of metastasis according to a radial basis machine (RBM) modeling algorithm. 5-yr MFS for the independent 104 sample validation set is 98% for class A compared to 79% for class B and 30% for class C (*p*<0.0001), with a 64% rate in the overall cohort, reflecting clinically valuable models.

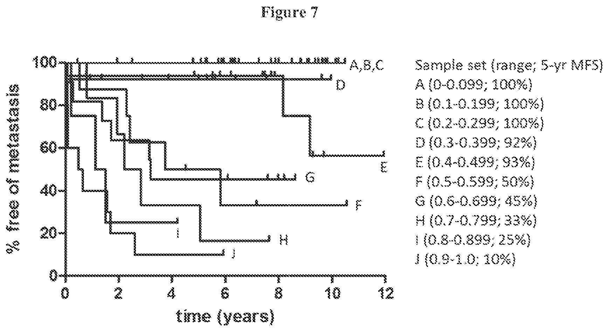

**Figure 7** depicts MFS for the 104 sample validation cohort based upon the probability score generated for each case during predictive modeling analysis. Subgroups A-J represent 0.1 unit incremental increases in the probability of metastasis (0=low risk of metastasis, 1= high risk of metastasis), as determined by the RBM algorithm. Importantly, increases in probability score correspond to decreases in MFS rates. For example, all cases in subgroup A have a probability score between 0 and 0.099, and there is 100% 5 year MFS in the cohort. Conversely, each of the cases is subgroup J has a probability score between 0.9 and 1.0, and none of those cases is metastasis free at the 5 year time point. Statistically significant differences are observed between the subgroups (*p*<0.0001), and 5-year MFS is shown in the figure legend.

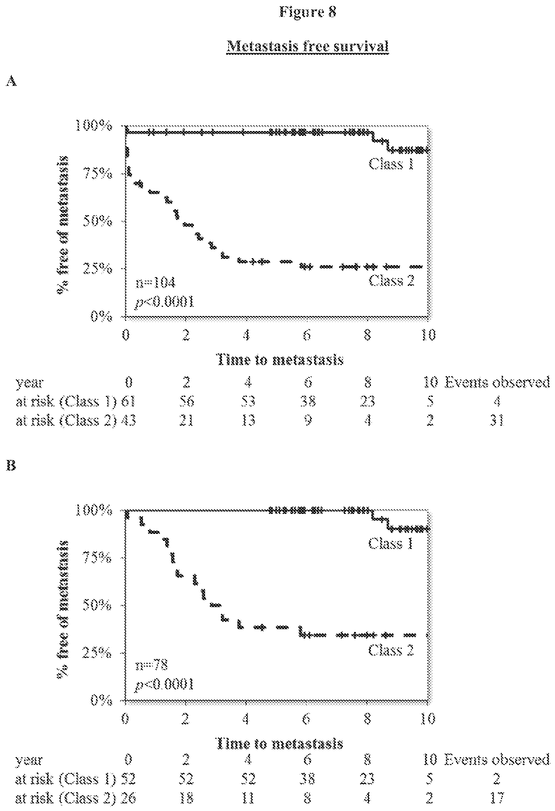

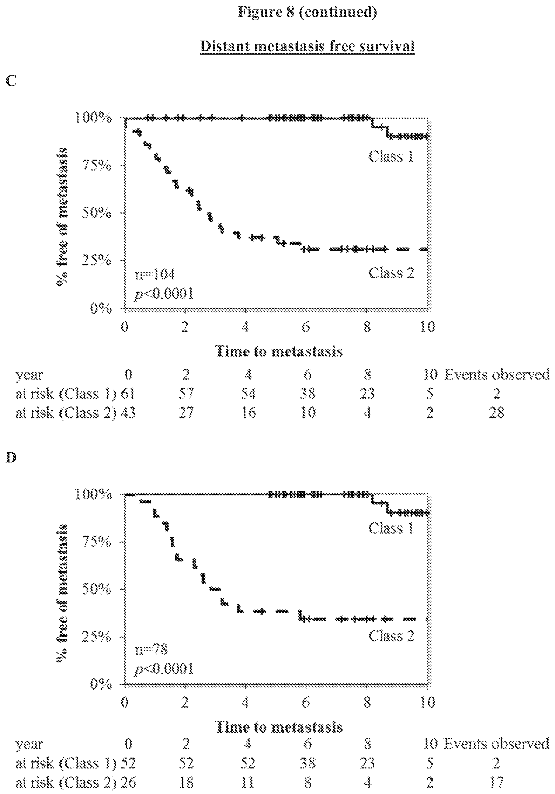

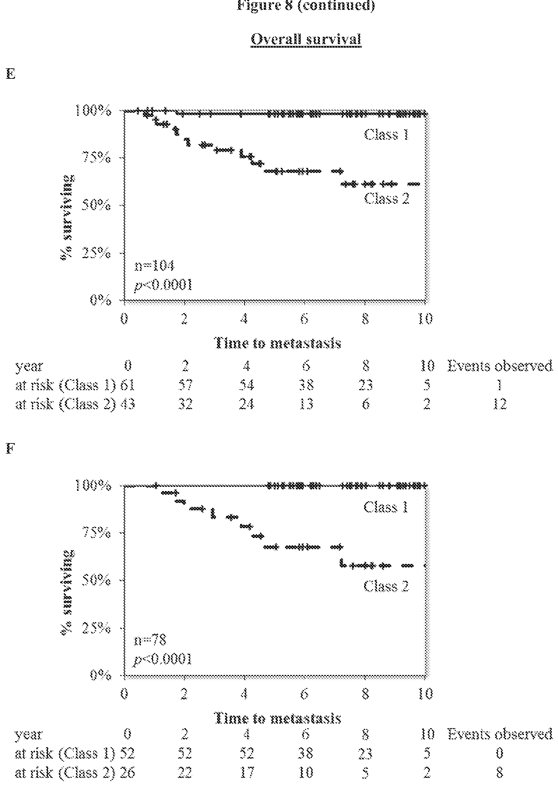

**Figure 8** depicts Kaplan-Meier analysis of metastasis-free survival, distant metastasis-free survival, and overall survival for a 104 case cohort that includes CM Stages I - IV (**A-C**), and for a 78 case cohort that includes only Stage I and II cases with evidence of metastasis, or no evidence of metastasis and greater than 5 years of follow-up (**D-E**).

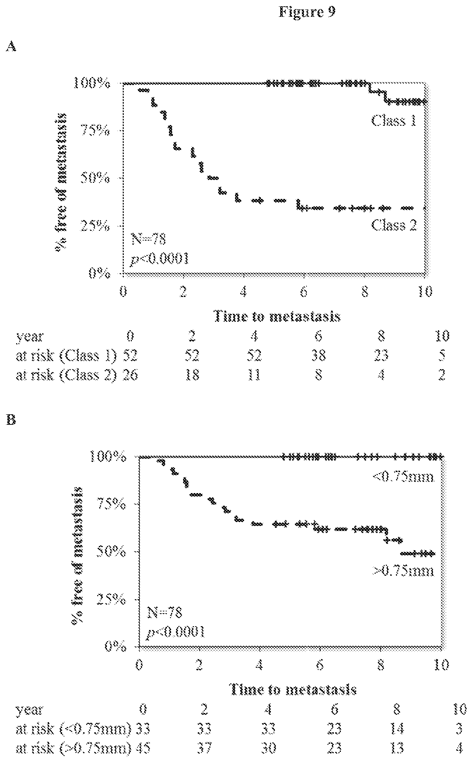

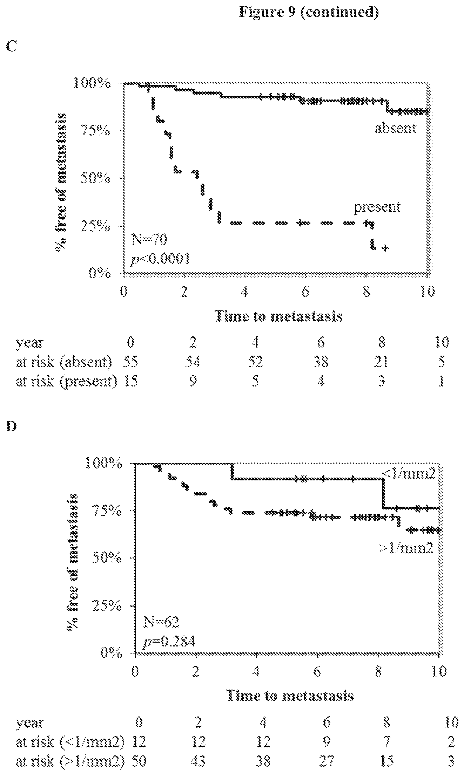

**Figure 9** depicts Kaplan-Meier analysis of distant metastasis-free survival (DMFS) comparing gene expression profile (GEP) to American Joint Committee on Cancer T-factors. DMFS is grouped by GEP class assignment **(A),** Breslow thickness of greater or less than 0.75mm **(B),** presence or absence of ulceration **(C),** or mitotic rate of greater or less than 1/mm² **(D).** N reflects the number of cases analyzed for each factor, based upon the availability of clinical data for those cases, and statistical significance.

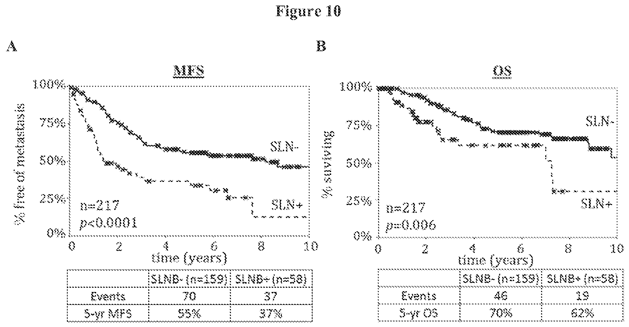

**Figure 10** depicts the metastasis free (MFS) and overall (OS) survival in a cohort of cutaneous melanoma primary tumor specimens collected from patients who underwent a SLNB procedure in the course of clinical management of the disease. Cases within the group are stratified according to those who had a negative SLN (SLN-; that is remained stage I or II under current definitions) or positive SLN (SLN+; that is were upstaged to stage III under current definitions) outcome following SLNB to determine the risk of metastatic disease. Of note, 70 SLN- patients developed metastatic disease and 46 died, resulting in 5-year MFS and OS of only 55% and 70%, respectively, compared to 37% and 62%, respectively, for patients with a SLN+ outcome.

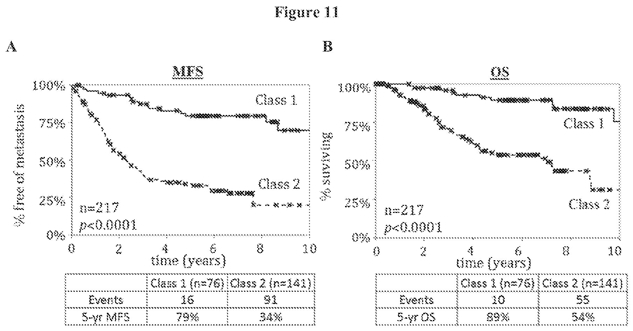

**Figure 11** depicts the metastasis free (MFS) and overall (OS) survival in a cohort of cutaneous melanoma primary tumor specimens collected from patients who underwent a SLNB procedure in the course of clinical management of their disease. Cases within the group are stratified according to those who had a Class 1 or Class 2 outcome following 28-gene GEP analysis to determine the risk of metastatic disease. Of note, only 16 of 76 Class 1 patients developed metastatic disease and 10 died of their disease. 5-year MFS and OS was 79% and 89%, respectively, for GEP Class 1 patients, compared to 55% and 70%, respectively, for patients with a SLN- outcome. Class 2 cases had similar 5-year MFS and OS (34% and 54%, respectively) compared to SLN+ cases (37% and 62%, respectively).

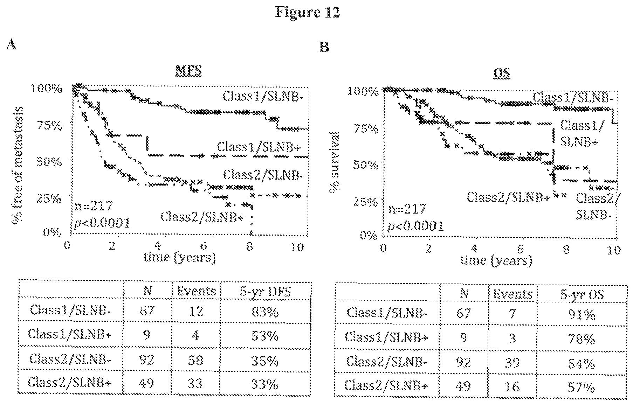

**Figure 12** depicts metastasis free (MFS) and overall (OS) survival in a cohort of cutaneous melanoma primary tumor specimens collected from patients who underwent a SLNB procedure in the course of clinical management of their disease. Cases within the group are stratified according to the combined outcomes predicted using the 28-gene GEP in combination with SLNB. Class 1/SLN- cases have the best prognosis for both MFS (83%) and OS (91%). Of significance, Class 2 patients with either a SLN- or SLN+ result have highly similar MFS (35% vs. 33%, respectively) and OS (54% vs. 57%, respectively), indicating that the GEP prognosis is more accurate than SLNB prognosis in high risk patients.

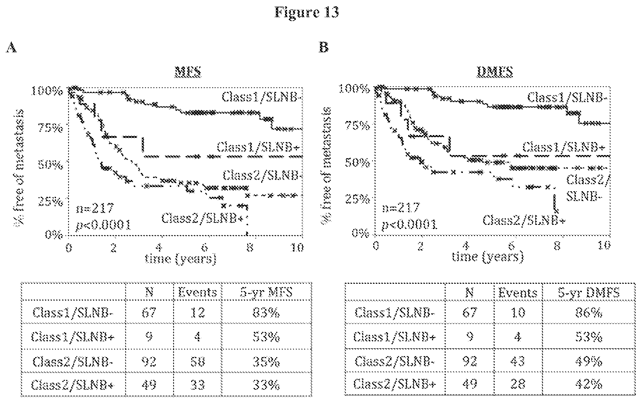

**Figure 13** depicts the comparison of metastasis free (MFS) and distant metastasis free (DMFS) survival in a cohort of cutaneous melanoma primary tumor specimens collected from patients who underwent a SLNB procedure in the course of clinical management of their disease. MFS includes metastasis within the nodal basin (regional recurrence and in transit disease), while DMFS includes only metastasis beyond the nodal basin. Cases within the group are stratified according to the combined outcomes predicted using the 28-gene GEP in combination with SLNB. Class 1/SLN- cases have the best prognosis for both MFS (83%) and DMFS (86%). The comparison of MFS and DMFS highlight the utility of GEP for predicting regional recurrence, and also reflect the value of GEP in combination with SLNB for predicting distant metastasis in CM patients.

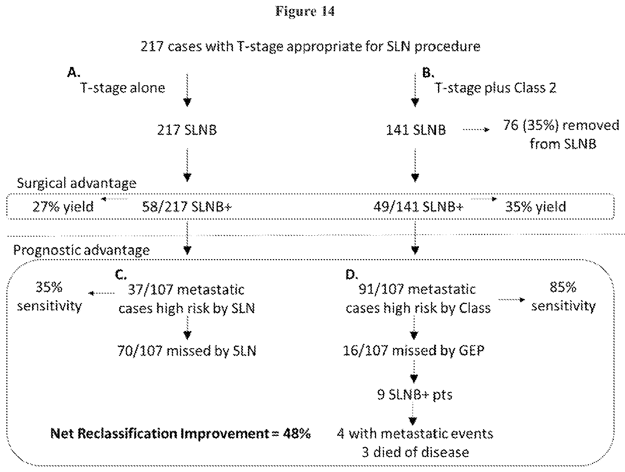

**Figure 14** depicts the surgical and prognostic advantages of including the GEP signature with AJCC T-stage factors and SLNB. Using T-stage alone (A), 217 CM cases were provided with a SLNB procedure. In this cohort, 58 cases were had a SLNB+ outcome, reflecting a 27% surgical yield. Addition of the GEP signature to T-factors **(B)** would have potentially reduced the number of SLNB procedures from 217 to 141, removing 76 (35%) cases, and identifying 49 cases with a SLNB+ outcome. Therefore, adding the GEP signature to AJCC staging provided a surgical yield of 35% (49/141) compared to 27% (58/217) with AJCC staging alone. A prognostic advantage was also observed for the GEP, as SLNB procedure **(C)** identified 37/107 cases (sensitivity = 35%) with a documented metastatic event, while adding GEP to SLNB **(D)** identified 91/107 cases (sensitivity = 85%) with a documented metastatic event. Of the 16 metastatic cases called Class 1 by the GEP signature, 9 had a SLN+ outcome. Of the 9, the GEP signature misclassified only 4 cases (1.8%) that had a distant metastatic event, and 3 cases (1.4%) that died from their disease. Overall, the GEP signature provided a net reclassification improvement of 48% in the 217 sample cohort of CM cases.

### DETAILED DESCRIPTION OF THE INVENTION

Unless otherwise defined, all technical and scientific terms used herein have the same meaning as commonly understood by one of ordinary skill in the art to which the invention belongs. Although methods and materials similar or equivalent to those described herein can be used to practice the invention, suitable methods and materials are described below. In case of conflict, the present specification, including definitions, will control. In addition, the materials, methods, and examples are illustrative only and are not intended to be limiting. Other features and advantages of the invention will be apparent from the following detailed description. Applicants reserve the right to alternatively claim any disclosed invention using the transitional phrase "comprising," "consisting essentially of," or "consisting of," according to standard practice in patent law.

Methods well known to those skilled in the art can be used to construct genetic expression constructs and recombinant cells according to this invention. These methods include *in vitro* recombinant DNA techniques, synthetic techniques, *in vivo* recombination techniques, and polymerase chain reaction (PCR) techniques. See, for example, techniques as described in Maniatis et al., 1989, MOLECULAR CLONING: A LABORATORY MANUAL, Cold Spring Harbor Laboratory, New York; Ausubel et al., 1989, CURRENT PROTOCOLS IN MOLECULAR BIOLOGY, Greene Publishing Associates and Wiley Interscience, New York, and PCR Protocols: A Guide to Methods and Applications (Innis et al., 1990, Academic Press, San Diego, CA).

Before describing the present invention in detail, a number of terms will be defined. As used herein, the singular forms "a", "an", and "the" include plural referents unless the context clearly dictates otherwise. For example, reference to a "nucleic acid" means one or more nucleic acids.

It is noted that terms like "preferably", "commonly", and "typically" are not utilized herein to limit the scope of the claimed invention or to imply that certain features are critical, essential, or even important to the structure or function of the claimed invention. Rather, these terms are merely intended to highlight alternative or additional features that can or cannot be utilized in a particular embodiment of the present invention.

For the purposes of describing and defining the present invention it is noted that the term "substantially" is utilized herein to represent the inherent degree of uncertainty that can be attributed to any quantitative comparison, value, measurement, or other representation. The term "substantially" is also utilized herein to represent the degree by which a quantitative representation can vary from a stated reference without resulting in a change in the basic function of the subject matter at issue.

As used herein, the terms "polynucleotide", "nucleotide", "oligonucleotide", and "nucleic acid" can be used interchangeably to refer to nucleic acid comprising DNA, RNA, derivatives thereof, or combinations thereof.

The inventors reviewed the current state of the art for evidence of microarray analyses focused on distinguishing differential genetic expression profiles that characterize cutaneous melanoma tumors. Seven studies were identified that utilized microarray technology to determine genetic expression comparing various stages of cutaneous melanoma.^{22-27,29} The objective was to identify genes that were dysregulated between radial growth phase to vertical growth phase, or in primary melanomas compared to metastatic tumors. Mauerer *et al.,* assessed gene expression differences in melanocytic nevi compared to primary melanoma, melanocytic nevi compared to metastatic melanoma, and primary melanoma compared to metastatic melanoma²⁵. Nothing in the art was found relating to evaluation of primary cutaneous melanoma tumors relative to subsequent metastatic versus non-metastatic outcomes. In an attempt to arrive at a putative gene expression profile set, the inventors focused on genes isolated from primary melanoma tumor samples and metastatic melanoma tumor samples that had an observed up-regulation greater than 2-fold, or down-regulation greater than 3-fold, and established those as potential mediators of metastatic progression. By these criteria, 26 up-regulated genes and 78 down-regulated genes were chosen as the basis for comparison to other expression analysis studies. This 104-gene panel was subsequently compared to expression data sets reported in Scatolini *et al.,* Jaeger, *et al.,* Winnipenninckx *et al.,* Haqq, *et al.,* Smith *et al.,* and Bittner *et al.*^{22-24,26,27,29} Additionally, the expression data from Onken, *et al.,* which reported 74 genes that were differentially regulated in metastatic and non-metastatic uveal melanoma tumors was compared to the 104-gene panel.¹⁴

The invention as disclosed herein is a method for predicting risk of metastasis, overall survival, or both, in a patient with a primary cutaneous melanoma tumor, the method comprising: (a) measuring gene expression levels of at least eight genes selected from the group consisting of BAP1_varA, BAP1_varB, MGP, SPP1, CXCL14, CLCA2, S100A8, BTG1, SAP130, ARG1, KRT6B, GJA, ID2, EIF1B, S100A9, CRABP2, KRT14, ROBO1, RBM23, TACSTD2, DSC1, SPRR1B, TRIM29, AQP3, TYRP1, PPL, LTA4H, and CST6, in a sample of the primary cutaneous melanoma tumor, wherein the at least eight genes are SPP1, MGP, KRT6B, PPL, RBM23, AQP3, CXCL14 and GJA1, wherein measuring gene expression levels of the at least eight genes comprises measurement of a level of fluorescence by a sequence detection system following RT-PCR of the at least eight genes; (b) determining a patient gene-expression profile signature comprising the gene expression levels of the at least eight genes; (c) comparing the patient gene-expression profile signature to a gene-expression profile of a predictive training set; and (d) providing an indication as to a risk of metastasis, overall survival or both for the primary cutaneous melanoma tumor when the patient gene expression profile indicates that the expression levels of at the least eight genes are altered in a predictive manner as compared to the gene expression profile of the predictive training set.

In an embodiment, the risk of metastasis for the primary cutaneous melanoma tumor is classified from a low risk of metastasis to a high risk of metastasis (for example, the tumor has a graduated risk from low risk to high or high to low risk of metastasis). In other embodiments, low risk of metastasis refers to 5-yr metastasis free survival rates of greater than 50%, 55%, 60%, 65%, 70%, 75%, 80%, 85%, 90%, 95% or more, and high risk of metastasis refers to a 5-yr metastasis free survival rates of less than 50%, 45%, 40%, 35%, 30%, 25%, 20%, 15%, 10%, 5% or less. In yet another embodiment, class 1 indicates that the tumor is at a low risk of metastasis, class 2 indicates that the tumor is at a high risk of metastasis, class A indicates that the tumor is at a low risk of metastasis, class B indicates that the tumor is at an intermediate risk of metastasis and class C indicates that the tumor is at a high risk of metastasis.

In some embodiments, the methods described herein can comprise determining that the primary cutaneous melanoma tumor has an increased risk of metastasis or decreased overall survival by combining with TNM (Tumor-Node-Metastasis) status, clinical staging set by American Joint Committee on Cancer (AJCC) to stage the primary cutaneous melanoma tumor, or by combining with sentinel lymph node biopsy status, or all three. In other embodiments, (1) Breslow thickness of greater or less than 0.75mm; or (2) presence or absence of ulceration; or (3) mitotic rate of greater or less than 1/mm²; or (4) sentinel lymph node biopsy status or any combination of the four can be used in combination with the gene-expression signature of a primary cutaneous melanoma. In an embodiment, a sentinel lymph node biopsy was performed in the patient from which the primary cutaneous melanoma tumor was separately obtained. In another embodiment the sentinel lymph node biopsy was negative.

Also disclosed but not forming part of the invention as disclosed herein is a method for treating cutaneous melanoma in a patient, the method comprising: (a) measuring gene expression levels of at least eight genes selected from the group consisting of BAP1_varA, BAP1_varB, MGP, SPP1, CXCL14, CLCA2, S100A8, BTG1, SAP130, ARG1, KRT6B, GJA, ID2, EIF1B, S100A9, CRABP2, KRT14, ROBO1, RBM23, TACSTD2, DSC1, SPRR1B, TRIM29, AQP3, TYRP1, PPL, LTA4H, and CST6 in a sample of a primary cutaneous melanoma tumor in the patient, wherein measuring gene expression levels of the at least eight genes comprises determining a level of fluorescence by a sequence detection system following RT-PCR of the at least eight genes; (b) determining a patient gene-expression profile signature comprising the gene expression levels of the at least eight genes; (c) comparing the patient gene-expression profile signature to a gene-expression profile of a predictive training set; (d) making a determination as to whether the patient gene-expression profile signature of the at least eight genes is altered in a predictive manner; and (e) administering an aggressive cancer treatment regimen to the patient when the determination is made in the affirmative that the patient has a primary cutaneous melanoma tumor with an increased risk of metastasis or decreased overall survival.

Also disclosed but not forming part of the invention as disclosed herein is a method of treating cutaneous melanoma in a patient, the method comprising: (a) measuring gene expression levels of at least eight genes selected from the group consisting of BAP1_varA, BAP1_varB, MGP, SPP1, CXCL14, CLCA2, S100A8, BTG1, SAP130, ARG1, KRT6B, GJA, ID2, EIF1B, S100A9, CRABP2, KRT14, ROBO1, RBM23, TACSTD2, DSC1, SPRR1B, TRIM29, AQP3, TYRP1, PPL, LTA4H, and CST6 in a sample of a primary cutaneous melanoma tumor in the patient, wherein measuring gene expression levels of the at least eight genes comprises determining a level of fluorescence by a sequence detection system following RT-PCR of the at least eight genes; (b) determining a patient gene-expression profile signature comprising the gene expression levels of the at least eight genes; (c) comparing the patient gene-expression profile signature to a gene-expression profile of a predictive training set; (d) making a determination as to whether the patient gene-expression profile signature of the at least eight genes is altered in a predictive manner; and (e) performing a sentinel lymph node biopsy (SLNB) on the patient when the determination is made in the affirmative that the patient has a primary cutaneous melanoma tumor with an increased risk of metastasis or decreased overall survival.

As used here in, "primary cutaneous melanoma tumor" refers to any primary melanoma lesion, regardless of tumor thickness, in patients without clinical or histologic evidence of regional or distant metastatic disease and which may be obtained through a variety of sampling methods such as punch biopsy, shave biopsy, wide local excision, and other means of extracting RNA from the primary melanoma lesion.

As used here in, "metastasis" is defined as the recurrence or disease progression that may occur locally (such as local recurrence and in transit disease), regionally (such as nodal micrometastasis or macrometastasis), or distally (such as brain, lung and other tissues). "Class 1 or class 2 of metastasis" as defined herein includes low-risk (class 1) or high-risk (class 2) of metastasis according to any of the statistical methods disclosed herein. Additionally, "cutaneous melanoma metastasis" as used herein includes sentinel lymph node metastasis, in transit metastasis, distant metastasis, and local recurrence.

As used herein, "overall survival" (OS) refers to the percentage of people in a study or treatment group who are still alive for a certain period of time after they were diagnosed with or started treatment for a disease, such as cancer. The overall survival rate is often stated as a five-year survival rate, which is the percentage of people in a study or treatment group who are alive five years after their diagnosis or the start of treatment.

The phrase "measuring the gene-expression levels" as used herein refers to determining or quantifying RNA or proteins expressed by the gene or genes. The term "RNA" includes mRNA transcripts, and/or specific spliced variants of mRNA. The term "RNA product of the gene" as used herein refers to RNA transcripts transcribed from the gene and/or specific spliced variants. In the case of "protein", it refers to proteins translated from the RNA transcripts transcribed from the gene. The term "protein product of the gene" refers to proteins translated from RNA products of the gene. A number of methods can be used to detect or quantify the level of RNA products of the gene or genes within a sample, including microarrays, RT-PCR (including quantitative RT-PCR), nuclease protection assays and Northern blot analyses. The assay may use the APPLIED BIOSYSTEMS^{™} HT7900 fast Real-Time PCR system. In addition, a person skilled in the art will appreciate that a number of methods can be used to determine the amount of a protein product of a gene of the invention, including immunoassays such as Western blots, ELISA, and immunoprecipitation followed by SDS-PAGE and immunocytochemistry.

A person skilled in the art will appreciate that a number of detection agents can be used to determine the expression of the genes. For example, to detect RNA products of the biomarkers, probes, primers, complementary nucleotide sequences or nucleotide sequences that hybridize to the RNA products can be used. To detect protein products of the biomarkers, ligands or antibodies that specifically bind to the protein products can be used.

The term "hybridize" refers to the sequence specific non-covalent binding interaction with a complementary nucleic acid. In an embodiment, the hybridization is under high stringency conditions. Appropriate stringency conditions which promote hybridization are known to those skilled in the art.

The term "probe" as used herein refers to a nucleic acid sequence that will hybridize to a nucleic acid target sequence. In one example, the probe hybridizes to an RNA product of the gene or a nucleic acid sequence complementary thereof. The length of probe depends on the hybridizing conditions and the sequences of the probe and nucleic acid target sequence. In one embodiment, the probe is at least 8, 10, 15, 20, 25, 50, 75, 100, 150, 200, 250, 400, 500 or more nucleotides in length.

As used herein, a "sequence detection system" is any computational method in the art that can be used to analyze the results of a PCR reaction. One example, *inter alia,* is the APPLIED BIOSYSTEMS^{™} HT7900 fast Real-Time PCR system. In certain embodiments, gene expression can be analyzed using, e.g., direct DNA expression in microarray, Sanger sequencing analysis, Northern blot, the NANOSTRING^{®} technology, serial analysis of gene expression (SAGE), RNA-seq, tissue microarray, or protein expression with immunohistochemistry or western blot technique.

As used herein the term "differentially expressed" or "differential expression" refers to a difference in the level of expression of the genes that can be assayed by measuring the level of expression of the products of the genes, such as the difference in level of messenger RNA transcript expressed or proteins expressed of the genes. In an embodiment, the difference is statistically significant. The term "difference in the level of expression" refers to an increase or decrease in the measurable expression level of a given gene as measured by the amount of messenger RNA transcript and/or the amount of protein in a sample as compared with the measurable expression level of a given gene in a control. In another embodiment, the differential expression can be compared using the ratio of the level of expression of a given gene or genes as compared with the expression level of the given gene or genes of a control, wherein the ratio is not equal to 1.0. For example, an RNA or protein is differentially expressed if the ratio of the level of expression in a first sample as compared with a second sample is greater than or less than 1.0. For example, a ratio of greater than 1, 1.2, 1.5, 1.7, 2, 3, 3, 5, 10, 15, 20 or more, or a ratio less than 1, 0.8, 0.6, 0.4, 0.2, 0.1, 0.05, 0.001 or less. In yet another embodiment the differential expression is measured using p-value. For instance, when using p-value, a biomarker is identified as being differentially expressed as between a first sample and a second sample when the p-value is less than 0.1, preferably less than 0.05, more preferably less than 0.01, even more preferably less than 0.005, the most preferably less than 0.001.

As used herein, the terms "control" and "standard" refer to a specific value that one can use to determine the value obtained from the sample. In one embodiment, a dataset may be obtained from samples from a group of subjects known to have a cutaneous melanoma type or subtype. The expression data of the genes in the dataset can be used to create a control (standard) value that is used in testing samples from new subjects. In such an embodiment, the "control" or "standard" is a predetermined value for each gene or set of genes obtained from subjects with cutaneous melanoma subjects whose gene expression values and tumor types are known.

As defined herein, "gene-expression profile signature" is any combination of genes, the measured messenger RNA transcript expression levels or direct DNA expression levels or immunohistochemistry levels of which can be used to distinguish between two biologically different corporal tissues and/or cells and/or cellular changes. The gene-expression profile signature is comprised of the gene-expression levels of at least 8 genes.

As defined herein, "predictive training set" means a cohort of CM tumors with known clinical metastatic outcome and known genetic expression profile, used to define/establish all other CM tumors, based upon the genetic expression profile of each, as a low-risk, class 1 tumor type or a high-risk, class 2 tumor type. Additionally, included in the predictive training set is the definition of "threshold points" points at which a classification of metastatic risk is determined, specific to each individual gene expression level.

As defined herein, "altered in a predictive manner" means changes in genetic expression profile that predict metastatic risk or predict overall survival. Predictive modeling risk assessment can be measured as: 1) a binary outcome having risk of metastasis or overall survival that is classified as low risk (*e.g.,* termed Class 1 herein) vs. high risk (*e.g.,* termed Class 2 herein); and/or 2) a linear outcome based upon a probability score from 0 to 1 that reflects the correlation of the genetic expression profile of a cutaneous melanoma tumor with the genetic expression profile of the samples that comprise the training set used to predict risk outcome. Within the probability score range from 0 to 1, a probability score, for example, less than 0.5 reflects a tumor sample with a low risk of metastasis or death from disease, while a probability score, for example, greater than 0.5 reflects a tumor sample with a high risk of metastasis or death from disease. The increasing probability score from 0 to 1 reflects incrementally declining metastasis free survival, as illustrated for example in Figure 7. For example, within subsets A, B and C in Figure 7, cutaneous melanoma tumors that have a probability score from 0 to 0.299 exhibit 5-year metastasis free survival rates are 100%, and rates remain above 90% for subsets D (0.3-0.399) and E (0.4-0.499). Conversely, tumor subsets F (0.5-0.599, 50% 5-yr MFS), G (0.6-0.699, 45% 5-yr MFS), H (0.7-0.799, 33% 5-yr MFS), I (0.8-0.899, 25% 5-yr MFS) and J (0.9-1.0, 10% 5-yr MFS), with probability scores between 0.5 and 1 exhibit significant decreases in 5-year survival rates with each 0.1 incremental increase in the probability score.

To develop a ternary, or three-class system of risk assessment, with Class A having a low risk of metastasis or death from disease, Class B having an intermediate risk, and Class C having a high risk, the median probability score value for all low risk or high risk tumor samples in the training set was determined, and one standard deviation from the median was established as a numerical boundary to define low or high risk. For example, as shown in Figure 6, low risk (Class A) cutaneous melanoma tumors within the ternary classification system have a 5-year metastasis free survival of 98%, compared to high risk (Class C) tumors with a 30% 5-year rate. Cases falling outside of one standard deviation from the median low or high risk probability scores have an intermediate risk, and intermediate risk (Class B) tumors have a 79% 5-year metastasis free survival rate.

The TNM (Tumor-Node-Metastasis) status system is the most widely used cancer staging system among clinicians and is maintained by the American Joint Committee on Cancer (AJCC) and the International Union for Cancer Control (UICC). Cancer staging systems codify the extent of cancer to provide clinicians and patients with the means to quantify prognosis for individual patients and to compare groups of patients in clinical trials and who receive standard care around the world. Clinical staging includes microstaging of the primary melanoma and clinical/radiologic evaluation for metastases. By convention, clinical staging should be used after complete excision of the primary melanoma with clinical assessment for regional and distant metastasis. The AJCC and the UICC update the TNM status cancer staging system periodically. The most recent revision is the 7th edition, effective for cancers diagnosed on or after January 1, 2010 (Edge SB, Byrd DR, Compton CC, Fritz AG, Greene FL, Trotti A, editors. AJCC cancer staging manual (7th ed). New York, NY: Springer; 2010, pages 325-344). Tumor-classification is summarized below in Table A.

**Table A. T-classification of melanoma of the skin**

| **T-CLASSIFICATION** | **THICKNESS (mm)** | **ULCERATION STATUS/MITOSES** |
|---|---|---|
| T1 | ≤1.0 | a: w/o ulceration and mitosis <1/mm² |
| | | b: with ulceration or mitoses ≥1/mm² |
| T2 | 1.01-2.0 | a: w/o ulceration |
| | | b: with ulceration |
| T3 | 2.01-4.0 | a: w/o ulceration |
| | | b: with ulceration |
| T4 | >4.0 | a: w/o ulceration |
| | | b: with ulceration |

As defined herein, "sentinel lymph node biopsy" refers is a procedure in which the first lymph node(s) (*i.e.,* the sentinel lymph node) to which cancer cells are most likely to spread from a primary tumor are identified, removed, and examined to determine whether cancer cells are present. A sentinel lymph node biopsy (SLNB) can be used to help determine the extent, or stage, of cancer in the body. During the SLNB procedure, multiple SLN's are biopsied. A negative SLNB result can suggest that cancer has not developed the ability to spread to nearby lymph nodes or other organs. A positive SLNB result indicates that cancer is present in the sentinel lymph node and may be present in other nearby lymph nodes (called regional lymph nodes) and, possibly, other organs. As a surgical intervention, SLNB has a low yield with only 5% of patients with CM staged at 1b that undergo SLNB yielding a positive SLNB, and only 18% of stage II patients yielding a positive SLNB. SNLB can have adverse effects. The potential adverse effects of lymph node surgery include the following: risk of complications from general anesthesia, lymphedema, or tissue swelling; seroma, or the buildup of lymph fluid at the site of the surgery; numbness, tingling, low of motor function or pain at the site of the surgery; difficulty moving the affected body part; and infection. SLNB, like other surgical procedures, can cause short-term pain, swelling, and bruising at the surgical site and increase the risk of infection. In addition, some patients may have skin or allergic reactions to the blue dye used in SLNB. Another clinically significant harm is a false-negative biopsy result-that is, cancer cells are not seen in the sentinel lymph node although they may be present in the lymphatic system - perhaps they haven't reached the SLNB, were biologically cleared from the SLNB and may be present regional lymph nodes, spread hematogenously, or other parts of the body. A false-negative biopsy result under-stages the patient, resulting in under-treatment of the patient's true risk of developing metastatic disease and gives the patient and the doctor a false sense of security about the extent of cancer in the patient's body. As demonstrated by the MSLT-1 study and the AJCC guidelines, approximately twice as many patients who are SLNB negative will metastasize than those that are SLNB positive.

As defined herein, "aggressive cancer treatment regimen" is determined by a medical professional and can be specific to each patient. An aggressive cancer treatment regimen is defined by the National Comprehensive Cancer Network (NCCN), and has been defined in the NCCN Guidelines^{®} as including one or more of 1) intensified imaging (CT scan, PET/CT, MRI, chest X-ray), 2) discussion and/or offering of sentinel lymph node biopsy with subsequent partial or complete lymphadenectomy, 3) inclusion in ongoing clinical trials, and 4) therapeutic intervention with interferon alpha treatment and radiation to nodal basin. Guidelines for clinical practice are published in the National Comprehensive Cancer Network (NCCN Guidelines^{®} Melanoma version 2.2013 available on the World Wide Web at NCCN.org). Additional therapeutic options include, but are not limited to, injections of Bacille Calmette-Guerin (BCG) vaccine, interferon, or interleukin-2 (IL-2) directly into the melanoma; radiation therapy without chemotherapeutic intervention; or applying imiquimod (ALDARA^{®}) cream. For melanomas on an arm or leg, another option might be isolated limb perfusion (*i.e.,* infusing the limb with a heated solution of chemotherapy). Other possible treatments include targeted therapy, such as immunotherapy (*e.g.*, ipilimumab/YERVOY^{®}), chemotherapy (*e.g*., dacarbazine/DTIC^{®} and temozolomide/TEMODAR^{®}), or immunotherapy combined with chemotherapy (*i.e*., biochemotherapy).

As used herein, the terms treatment, treat, or treating refers to a method of reducing the effects of a disease or condition or symptom of the disease or condition. Thus, in the disclosed method, treatment can refer to a 5%, 10%, 20%, 30%, 40%, 50%, 60%, 70%, 80%, 90%, or 100% reduction in the severity of an established disease or condition or symptom of the disease or condition. For example, a method of treating a disease is considered to be a treatment if there is a 5% reduction in one or more symptoms of the disease in a subject as compared to a control. Thus, the reduction can be a 5%, 10%, 20%, 30%, 40%, 50%, 60%, 70%, 80%, 90%, 100% or any percent reduction between 5 and 100% as compared to native or control levels. It is understood that treatment does not necessarily refer to a cure or complete ablation of the disease, condition, or symptoms of the disease or condition.

In one embodiment, the cutaneous melanoma tumor is taken from a formalin-fixed, paraffin embedded wide local excision sample. In another embodiment, the cutaneous melanoma tumor is taken from formalin-fixed, paraffin embedded punch biopsy sample. In another embodiment, the cutaneous melanoma tumor is taken from formalin-fixed, paraffin embedded shave biopsy sample.

In certain embodiments, analysis of genetic expression and determination of outcome is carried out using radial basis machine and/or partition tree analysis, logistic regression analysis (LRA), K-nearest neighbor, or other algorithmic approach. These analysis techniques take into account the large number of samples required to generate a training set that will enable accurate prediction of outcomes as a result of cut-points established with an in-process training set or cut-points defined for non-algorithmic analysis, but that any number of linear and nonlinear approaches can produce a statistically significant and clinically significant result. Among the advantages of use of the methods disclosed herein are relating to, *e.g.,* the in excess of 140 samples in the training set used to cover either heterogeneity or adequately handle smaller gene expression profile changes that could not adequately predict outcomes in an independent test set. As defined herein, "Kaplan-Meier survival analysis" is understood in the art to be also known as the product limit estimator, which is used to estimate the survival function from lifetime data. In medical research, it is often used to measure the fraction of patients living for a certain amount of time after treatment. JMP GENOMICS^{®} software provides an interface for utilizing each of the predictive modeling methods disclosed herein, and should not limit the claims to methods performed only with JMP GENOMICS^{®} software.

### EXAMPLES

The Examples that follow are for explanatory purposes only, and should not be construed as limiting the scope of the invention in any way.

### Materials and Methods

### 1. Cutaneous melanoma tumor sample preparation and RNA isolation

Formalin fixed paraffin embedded (FFPE) primary cutaneous melanoma tumor specimens arranged in 5µm sections on microscope slides were acquired from multiple institutions under Institutional Review Board (IRB) approved protocols. All tissue was reviewed by a pathologist to confirm the presence of melanoma and the dissectible tumor area was marked. Tumor tissue was dissected from the slide using a sterile disposable scalpel, collected into a microcentrifuge tube, and deparaffinized using xylene. RNA was isolated from each specimen using the Ambion RECOVERALL^{™} Total Nucleic Acid Isolation Kit (Life Technologies Corporation, Grand Island, NY). RNA quantity and quality were assessed using the NANODROP^{™} 1000 system and the Agilent Bioanalyzer 2100.

### 2. cDNA generation and RT-PCR analysis

RNA isolated from FFPE samples was converted to cDNA using the APPLIED BIOSYSTEMS^{™} High Capacity cDNA Reverse Transcription Kit (Life Technologies Corporation, Grand Island, NY). Prior to performing the RT-PCR assay each cDNA sample underwent a 14-cycle pre-amplification step. Pre-amplified cDNA samples were diluted 20-fold in TE buffer. 50ul of each diluted sample was mixed with 50ul of 2X TAQMAN^{®} Gene Expression Master Mix, and the solution was loaded to a custom high throughput microfluidics gene card containing primers specific for 28 class discriminating genes and 3 endogenous control genes (HNRNPL, YKT2 and FXR1). Each sample was run in triplicate. The gene expression profile test was performed on an APPLIED BIOSYSTEMS^{™} HT7900 machine (Life Technologies Corporation, Grand Island, NY).

### 3. Expression analysis and class assignment

Mean Cₜ values were calculated for triplicate sample sets, and ΔCₜ values were calculated by subtracting the mean Cₜ of each discriminating gene from the geometric mean of the mean Cₜ values of all three endogenous control genes (HNRNPL, YKT2 and FXR1). ΔCₜ values were standardized according to the mean of the expression of all discriminant genes with a scale equivalent to the standard deviation. Three control genes were selected based upon analysis using geNorm. Various linear and non-linear predictive modeling methods, including radial basis machine, k-nearest neighbor, partition tree, logistic regression, discriminant analysis and distance scoring, were performed using JMP GENOMICS^{®} SAS-based software (JMP, Cary, NC). Kaplan-Meier curves reflecting metastasis free survival were also generated in JMP, and statistical significance was calculated according to the Log Rank method. Cox univariate and multivariate regression analysis was performed using WinSTAT for Microsoft Excel version 2012.1.

### Example 1: Cutaneous melanoma metastatic risk genetic signature and biomarker expression

Genetic expression of the discriminant genes in the signature (Table 1) was assessed in a cohort of 268 cutaneous melanoma samples using RT-PCR (Figure 1). As shown in Table 2 below, of the 28 discriminating genes, 26 were significantly altered in metastatic melanoma tumors compared to nonmetastatic tumors (p < 0.05, range 0.0366-6.08E-16), and 25 were down-regulated. Genes that were up-regulated in the metastatic tumors included SPP1, KRT6B, and EIF1B.

**Table 1: Genes included in the GEP signature able to predict metastatic risk from primary CM tumors.**

| **Gene Symbol** | **Gene Name** | **Alternative Gene Names** |
|---|---|---|
| BAP1_varA | BRCA1 associated protein-1 | TPDS, UCHL2, HUCEP-13, HUCEP-6, BAP_var1, BAP (a1) |
| BAP1_varB | BRCA1 associated protein-1 | UCHL2, HUCEP-13, HUCEP-6, BAP_var2, BAP (a2) |
| MGP | matrix gamma-carboxyglutamic acid | matrix Gla protein, gamma-carboxyglutamic acid, GIG36, MGLAP, NTI |
| SPP1 | secreted phosphoprotein 1 | BNSP, BSPI, ETA-1, OPN, PSEC0156 |
| CXCL14 | chemokine (C-X-C motif) ligand 14 | small inducible cytokine subfamily B member 14, UNQ240/PRO273, BMAC, BRAK, KEC, KS1, MIP-2g, MIP2G, NJAC, SCYB14 |
| CLCA2 | chloride channel accessory 2 | chloride channel regulator 2, chloride channel calcium activated 2, CACC, CACC3, CLCRG2, CaCC-3 |
| S100A8 | S100 calcium binding protein A8 | calgranulin-A, calprotectin L1L subunit, cystic fibrosis antigen, leukocyte L1 complex light chain, migration inhibitory factor-related protein 8, urinary stone protein band A, protein S100-A8, 60B8AG, CAGA, CFAG, CGLA, CP-10, L1Ag, MA387, MIF, MRP8, NIF, P8 |
| S100A9 | S100 calcium binding protein A9 | calgranulin-B, calprotectin L1H subunit, leukocyte L1 complex heavy chain, migration inhibitory factor-related protein 14, 60B8AG, CAGB, CFAG, CGLB, L1AG, LIAG, MAC387, MIF, MRP14, NIF, P14 |
| BTG1 | B-cell translocation gene 1, antiproliferative | B-cell translocation gene 1 protein |
| SAP130 | Sin3A-associated protein, 130kDa | |
| ARG1 | arginase-1 | liver-type arginase, type I arginase |
| KRT6B | keratin 6B | keratin type II cytoskeletal 6B, cytokeratin-6B, type II keratin Kb10, CK-6B, CK6B, K6B, KRTL1, PC2 |
| GJA1 | gap junction protein, alpha 1 | connexin-43, gap junction 43 kDa heart protein, AU042049, AW546267, Cnx43, Cx43, Cx43alpha1, Gja-1, Npm1 |
| ID2 | inhibitor of DNA binding 2, dominant negative helix-loop-helix protein | class B basic helix-loop-helix protein, GIG8, ID2A, ID2H, Idb2, bHLHb26 |
| EIF1B | eukaryotic translation initiation factor 1B | protein translation factor SUI1 homolog GC20 |
| CRABP2 | cellular retinoic acid binding protein 2 | RP11-66D17.5, CRABP-II, RBP6 |
| KRT14 | keratin 14 | keratin type I cytoskeletal 14, cytokeratin-14, CK14, EBS3, EBS4, K14, NFJ |
| ROBO1 | roundabout, axon guidance receptor, homolog 1 (Drosophila) | roundabout homolog 1, deleted in U twenty twenty, H-Robo-1, DUTT1, SAX3 |
| RBM23 | RNA binding motif protein 23 | RNA-binding region-containing protein 4, splicing factor SF2, PP239, CAPERbeta, RNPC4 |
| TACSTD2 | tumor-associated calcium signal transducer 2 | cell surface glycoprotein Trop-2, membrane component chromosome 1 surface marker 1, pancreatic carcinoma marker protein GA733-1, GA733-1, TROP2, EGP-1, EGP1, GA7331, GP50, M1S1 |
| DSC1 | desmocollin 1 | cadherin family member 1, desmosomal glycoprotein 2/3, CDHF1, DG2/DG3 |
| SPRR1B | small proline-rich protein 1B | CORNIFIN, cornifin-B, 14.9 kDa pancornulin, GADD33, SPRR1 |
| TRIM29 | tripartite motif containing 29 | ATDC |
| AQP3 | aquaporin 3 | aquaglyceroporin-3, AQP-3, GIL |
| TYRP1 | tyrosinase-related protein 1 | RP11-3L8.1, CAS2, CATB, GP75, OCA3, TRP, TRP1, TYRP, b-PROTEIN |
| PPL | periplakin | 190 kDa paraneoplastic pemphigus antigen, 195 kDa cornified envelope precursor protein |
| LTA4H | leukotriene A4 hydrolase | LTA-4 hydrolase |
| CST6 | cystatin E/M | |

**Table 2: Genes included in the GEP signature able to predict metastatic risk from primary CM tumors.**

| | **Delta-Ct value** | | | | |
|---|---|---|---|---|---|
| **Gene symbol** | **nonmetastatic samples** | **metastatic samples** | **expression change in metastatic samples** | **p-value** | **direction of expression change** |
| BAP1 varA | -1.290 | -1.677 | -0.388 | 0.007118 | down |
| MGP | **-1.996** | -2.190 | -0.194 | 0.48585 | down |
| SPP1 | -1.011 | 2.224 | 3.235 | 6.08E-16 | up |
| CXCL14 | 3.021 | 0.828 | -2.193 | 3.31E-12 | down |
| BAP1_varB | 0.381 | 0.003 | -0.378 | 0.004646 | down |
| CLCA2 | -3.468 | -5.603 | -2.135 | 1.02E-08 | down |
| S100A8 | -0.450 | -1.179 | -0.728 | 0.030655 | down |
| BTG1 | -2.422 | -3.008 | -0.586 | 0.023606 | down |
| SAP130 | -1.075 | -1.405 | -0.329 | 0.023626 | down |
| ARG1 | -1.645 | -4.393 | -2.749 | 1.05E-08 | down |
| KRT6B | -1.809 | -1.222 | 0.586 | 0.160458 | up |
| GJA1 | -2.882 | -3.652 | -0.770 | 0.034149 | down |
| ID2 | -0.649 | -1.411 | -0.762 | 3.91E-06 | down |
| EIF1B | 0.041 | 0.350 | 0.309 | 0.023747 | up |
| S100A9 | 3.374 | 2.527 | -0.847 | 0.012385 | down |
| CRABP2 | -0.087 | -0.953 | -0.866 | 0.00059 | down |
| KRT14 | 5.654 | 3.927 | -1.727 | 1.75E-05 | down |
| ROBO1 | 0.100 | -0.364 | -0.464 | 0.000406 | down |
| RBM23 | -2.788 | -3.161 | -0.374 | 0.018025 | down |
| TACSTD2 | -3.485 | -3.984 | -0.499 | 0.03658 | down |
| DSC1 | -0.102 | -2.963 | -2.861 | 7E-09 | down |
| SPRR1B | 4.622 | 3.139 | -1.482 | 0.001392 | down |
| TRIM29 | 0.228 | -2.239 | -2.467 | 2.34E-09 | down |
| AQP3 | 3.413 | 1.848 | -1.565 | 5.08E-06 | down |
| TYRP1 | 1.276 | -0.850 | -2.125 | 2.41E-06 | down |
| PPL | -0.082 | -2.233 | -2.150 | 5.59E-11 | down |
| LTA4H | -0.736 | -1.275 | -0.539 | 0.000156 | down |
| CST6 | -0.535 | -3.099 | -2.563 | 1.02E-08 | down |

### Example 2: Initial training set development studies and comparison to validation cohort

Using JMP GENOMICS^{®} software and clinical data analysis, a training set of 164 cutaneous melanoma samples was generated that could accurately predict the risk of metastasis based upon the 28 gene signature. The training set contained 15 stage 0 *in situ* melanomas, 61 stage I, 70 stage II, 17 stage III, and 1 stage IV melanomas. Metastatic risk was assessed using a radial basis machine predictive modeling algorithm, which reports class 1 (low risk of metastasis) or class 2 (high risk of metastasis). ΔCt values generated from RT-PCR analysis of the training set cohort were standardized to the mean for each gene, with a scale equivalent to the standard deviation. Analyses were also performed using KNN, PTA, and discriminant analysis to confirm the results from the RBM approach (as discussed below). The training set prediction algorithm was then validated using an independent cohort of 104 cutaneous melanoma samples, comprised of 57 stage I, 34 stage II, 11 stage III, and 2 stage IV melanomas. Samples in the validation set were standardized prior to analysis using the factorials generated during standardization of the training set. Area under the receiver operator characteristic (ROC) curve, accuracy, sensitivity (prediction of high risk metastatic event), and specificity (prediction of nonmetastatic outcome) were statistical endpoints for the analysis. In the training set cohort, ROC = 0.9052, accuracy = 83%, sensitivity = 85%, and specificity =80% (Figure 1A). In the validation cohort, ROC = 0.9089, accuracy = 86%, sensitivity = 89%, and specificity = 83% (Figure 1B).

Kaplan-Meier survival analysis was also performed for the training and validation cohorts. In the training set, 5-year metastasis free survival (MFS) was 91% for class 1 cases, and 25% for class 2 cases (p<0.0001; Figure 1A). By comparison, 5-year MFS for the validation cohort was 97% for class 1 cases and 31% for class 2 cases (p<0.0001; Figure 1B). Overall, the 5-year MFS rate for the entire training set, combining class 1 and class 2 cases, was 64%, while the combined MFS rate for the validation set was 69%.

### Example 3: Analysis of 162 sample training set with multiple predictive modeling methods

JMP GENOMICS^{®} software allows for analysis using linear and non-linear predictive modeling methods. To assess whether accuracy of metastatic risk prediction for the validation cohort was limited to the RBM method, partition tree, K-nearest neighbor, logistic regression, and discriminant analysis were performed (Figure 2 and 3). Training set ROC, accuracy, sensitivity, specificity and K-M 5-year MFS for class 1 and class 2 cases were highly comparable to the RBM method. Highly accurate prediction of metastasis, and significantly different 5-year MFS between class 1 and class 2 cases was observed when using partition tree (Figure 2A), K-nearest neighbor (Figure 2B), logistic regression (Figure 2C), or discriminant analysis (Figure 2D). Significant differences in 5-year MFS were also observed for validation cohort class 1 and class 2 cases using partition tree (Figure 3A), K-nearest neighbor (Figure 3B), logistic regression (Figure 3C), and discriminant analysis (Figure 3D). Importantly, though, accuracy of prediction for validation samples was above 80% with all methods except partition tree analysis (Figure 3A), and sensitivity, or accuracy of prediction for cases with documented metastatic events, was as high as 91% when using logistic regression or discriminant analysis.

### Example 4: Evaluation of in situ melanoma effect upon training set accuracy

To assess the impact of stage 0 *in situ* melanoma samples on the predictive capabilities of the training set, either a) samples were removed from all stage 0 the training and validation cohorts, generating a new training set of 149 cases; or b) all stage 0 samples were included in the training set only, generating a training set comprised of 164 cases. The new cutaneous melanoma predictive training sets were used to train a 104 sample validation cohort.

Radial basis machine prediction was performed as described above, and ROC, accuracy of prediction, and 5-year MFS were assessed for the training and validation sets (Figure 4). Training set statistics for both the 149 sample and 164 sample training sets (Figure 4A and 4B) were highly comparable. ROC was highest for the 149 sample set, but accuracy, sensitivity and specificity were highly similar in the 149 and 164 sample training sets. K-M analysis yielded highly significant differences between class 1 and class 2 MFS with each training set. Metastatic risk of the 104 samples in the validation cohort was accurately predicted with the 149 and 164 sample training sets (Figure 4C and 4D). Both validation sets had ROC greater than 0.9, reflecting highly relevant clinical models of prediction, and sensitivity of 83% and 89% when class prediction was done using the 149 and 164 sample training sets, respectively. Again, MFS was significantly different between class 1 and class 2 regardless of the training set cohort used to predict risk in the validation set.

### Example 5: Identification of reduced discriminant gene signatures with predictive accuracy

Accurate prediction of high risk metastatic cases is extremely important in order to prevent those patients who are likely to metastasize from receiving a low risk treatment protocol. Thus, a measure of success for the predictive gene set is achievement of greater than 88% sensitivity in both the training and validation sets. As shown in Table 3 below, and in Figure 4, sensitivity is 85% for the 164 sample training set and 89% for the 104 sample validation set when the 28-gene signature incorporating all discriminant genes is used to predict metastatic risk. Smaller sets of genes were generated that had equal or increased sensitivity compared to the 28-gene signature (Table 3, bolded). However, the majority of gene sets that did not include all 28 genes were not able to produce the sensitivity thresholds required for use in a clinically feasible GEP test.

**Table 3: Sensitivity, or accuracy of predicting a metastatic event, achieved when using the 28-gene signature or smaller subsets of genes.**

| **Gene set** | **# of variables** | **Sensitivity training/validation sets (%)** |
|---|---|---|
| All discriminant genes | 28 | 89/94 |
| SPP1, CXCL14, BAP1varB, CLCA2, S100A8, BTG1 | 6 | 79/89 |
| TACSTD2, RBM23, PPL, S100A8, MGP, TYRP1 | 6 | 86/78 |
| SAP130, ARG1, KRT6B, GJA1, EIF1B, ID2 | 6 | 81/81 |
| CRABP2, KRT14, ROBO1, RBM23, TACSTD2, DSC1 | 6 | 70/81 |
| ROBO1, CST6, BAP1varB, ID2, SPRR1B, KRT6B | 6 | 75/83 |
| SPRR1B, AQP3, PPL, DSC1,TYRP1, TRIM29 | 6 | 81/81 |
| **KRT6B, GJA1, AQP3, TRIM29, TYRP1, RBM23, MGP, EIF1B** | **8** | **93/94** |
| SPP1, MGP, KRT6B, PPL, RBM23, AQP3, CXCL14, GJA1 | 8 | 86/92 |
| BAP1_varB, S100A8, ARG1, S100A9, RBM23, DSC1, TYRP1, CST6 | 8 | 77/94 |
| BAP1_varA, BTG1, ARG1, GJA1, EIF1B, TACSTD2, TYRP1, LTA4H | 8 | 74/89 |
| GJA1, ID2, KRT14, ROBO1, RBM23, TRIM29, LTA4H, S100A9 | 8 | 70/69 |
| DSC1, SPRR1B, TRIM29, AQP3, TYRP1, PPL, LTA4H, CST6 | 8 | *72*/*86* |
| KRT14, ROBO1, RBM23, TACSTD2, DSC1, SPRR1B, TRIM29, AQP3 | 8 | 68/72 |
| BAP1_varA, MGP, SPP1, CXCL14, BAP1_varB, CLCA2, S100A8, BTG1 | 8 | 81/83 |
| SAP130, ARG1, KRT6B, GJA1, EIF1B, ID2, EIF1B, S100A9, CRABP2 | 8 | 84/81 |
| MGP, SAP130, GJA1, ID2, S100A9, ROBO1, AQP3, LTA4H | 8 | 65/69 |
| **SAP130, ARG1, KRT6B, EIF1B, S100A9, KRT14, ROBO1, RBM23, TRIM29, AQP3, TYRP1, CST6** | **12** | **91/97** |
| **GJA1, PPL, ROBO1, MGP, TRIM29, AQP3, RBM23, TACSTD2, TYRP1, KRT6B, EIF1B, DSC1** | **12** | **93/94** |
| **CRABP2, TYRP1, PPL, EIF1B, SPRR1B, DSC1, GJA1, AQP3, MGP, RBM23, CLCA2, TRIM29** | **12** | **93/89** |
| **RBM23, TACSTD2, CRABP2, PPL, GJA1, SPP1, CXCL14, EIF1B, AQP3, MGP, LTA4H, KRT6B** | **12** | **91/89** |
| BAP1_varA, SPP1, BAP1_varB, S100A8, SAP130, KRT6B, ID2, S100A9, ROBO1, TACSTD2 | 12 | 86/83 |
| BAP1_varA, MGP, S100A8, BTG1, ARG1, S100A9, KRT14, ROBO1, SPRR1B, TRIM29, AQP3, LTA4H | 12 | 75/75 |
| SPP1, BAP1_varB, CLCA2, SAP130, GJA1, S100A9, RBM23, SPRR1B, TYRP1, BTG1, KRT6B | 12 | 84/89 |
| EIF1B, S100A9, CRABP2, KRT14, ROBO1, RBM23, TRIM29, AQP3, TYRP1, PPL, LTA4H, CST6 | 12 | 82/89 |
| CXCL14, BAP1_varB, CLCA2, S100A8, BTG1, SAP130, S100A9, CRABP2, KRT14, ROBO1, RMB23, TACSTD2 | 12 | *79*/*92* |
| MGP, CLCA2, S100A8, ARG1, GJA1, ID2, S100A9, ROBO1, RBM23, SPRR1B, TRIM29, AQP3 | 12 | 70/78 |
| **S100A8, TACSTD2, BAP1_varA, KRT6B, EIF1B, TRIM29, TYRP1, CST6, PPL, RBM23, AQP3, GJA1, SPRR1B, ARG1** | **14** | **91/89** |
| BAP1_varB, CLCA2, BTG1, SAP130, GJA1, ID2, S100A9, CRABP2, RBM23, TACSTD2, DSC1, LTA4H, SPP1, KRT6B | 14 | 86/89 |
| SPP1, CLCA2, S100A8, SAP130, ARG1, ID2, EIF1B, S100A9, KRT14, ROBO1, DSC1, TRIM29, TYRP1, LTA4H | 14 | 86/97 |
| CXCL14, BAP1_varB, S100A8, BTG1, ARG1, KRT6B, ID2, EIF1B, CRABP2, KRT14, RBM23, TACSTD2, SPRR1B, TRIM29 | 14 | 89/82 |
| BAP1_varA, MGP, SPP1, CXCL14, BAP1_varB, CLCA2, S100A8, BTG1, SAP130, ARG1, KRT6B, GJA1, ID1, EIF1B | 14 | 84/86 |
| S100A9, CRABP2, KRT14, ROBO1, RBM23, TACSTD2, DSC1, SPRR1B, TRIM29, AQP3, TYRP1, PPL, LTA4H, CST6 | 14 | 82/78 |
| BTG1, SAP130, ARG1, GJA1, EIF1B, CRABP2, ROBO1, RBM23, TACSTD2, DSC1, SPRR1B, AQP3, PPL, CST6 | 14 | 84/86 |
| BAP1_varA, MGP, BAP1_varB, CLCA2, BTG1, SAP130, GJA1, ID2, S100A9, CRABP2, RBM23, TACSTD2, DSC1, LTA4H | 14 | 72/72 |
| MGP, CXCL14, S100A8, BTG1, ARG1, GJA1, ID2, S100A9, KRT14, ROBO1, TACSTD2, SPRR1B, TRIM29, TYRP1 | 14 | 82/86 |
| S100A8, BTG1, SAP130, EIF1B, S100A9, CRABP2, KRT14, DSC1, SPRR1B, TRIM29, AQP3, CST6, BAP1varB, LTA4H | 14 | 79/86 |
| **CST6, KRT6B, LTA4H, CLCA2, CRABP2, TRIM29, CXCL14, PPL, ARG1, RBM23, GJA1, AQP3, TYRP1, SPP1, DSC1, TACSTD2, EIF1B, BAP1_varA** | **18** | **91/89** |
| MGP, CXCL14, CLCA2, BTG1, ARG1, GJA1, EIF1B, CRABP2, ROBO1, TACSTD2, DSC1, SPRR1B, TRIM29, AQP3, TYRP1, PPL, LTA4H, CST6 | 18 | 81/92 |
| MGP, BAP1_varB, CLCA2, S100A8, BTG1, KRT6B, GJA1, ID2, EIF1B, S100A9, ROBO1, RBM23, TACSTD2, AQP3, TYRP1, PPL, LTA4H, SPRR1B | 18 | 81/100 |

### Example 6: 28-gene signature for cutaneous melanoma predicts aggressive tumor progression to SLN, distant lymph nodes, in transit metastasis, distant metastasis, and local recurrence

A total of 29 stage III, sentinel lymph node (SNL) positive, samples were included in the study population (17 in the training set and 12 in the validation set). These results are shown below in Table 4. RBM prediction of risk for the stage III samples resulted in 25 of 29 (86%) cases classified as high risk, class 2. Metastasis beyond the SLN was documented for 23 of the 29 cases, and 21 of those were predicted to be class 2 by the algorithm. Metastatic events localized to distant lymph nodes were documents for 13 cases, 12 (92%) of which were accurately predicted to be class 2. Similarly, 6 of 7 (86%) of in transit metastasis cases were assigned to class 2, indicating that the 28-gene predictive signature accurately predicts all aggressive cutaneous melanoma tumor types, including SLN positive, distant nodal, in transit, distant visceral metastases and local recurrence.

**Table 4: Accuracy of the CM 28-gene signature for predicting metastatic risk in SLN, distant lymph nodes, in transit metastasis, and locally recurrent disease.**

| | | **Training set** | | **Validation set** | |
|---|---|---|---|---|---|
| | | **total** | **called class 2** | **total** | **called class 2** |
| SLN+ (stage III) samples | | 17 | 14 (82%) | 12 | 11 (92%) |
| | documented met event | 12 | 11 (92%) | 11 | 10 (91%) |
| | no documented met event | 5 | 3 (60%) | 1 | 1 (100%) |
| distant lymph node metastasis | | 6 (2 SLN+) | 6 (100%) | 7 (5 SLN+) | 6 (86%) |
| in transit metastasis | | 3 | 2 (66%) | 4 | 4 (100%) |
| local recurrence | | 9 | 4* (44%) | 5** | 3 (60%) |
| *2/4 LR have known metastatic event (both called class 2) | | | | | |
| **1/5 LR is stage III (SLN+) - called class 2 | | | | | |

### Example 7: Statistical comparison of GEP signature to common CM prognostic factors

Cox univariate and multivariate regression analysis was performed for the 104 sample validation cohort following prediction of metastatic risk (Tables 5 and 6). GEP was compared to the individual prognostic factors comprising AJCC pathologic stage, including Breslow thickness, mitotic rate, and ulceration status, and was also compared directly to AJCC stage. AJCC stage was analyzed as known in the art as Stage IA, IB, IIA vs. IIB, IIC, III, IV for the complete validation set, and as Stage IA, IB, IIA vs. IIB, IIC for analysis that included only Stage I and II. Thus, analysis was performed with inclusion of all validation set cases (Table 5), or with inclusion of only stage I and stage II cases (Table 6). Cox univariate analysis resulted in significant correlation with metastasis for all factors except mitotic rate regardless of whether all validation set cases were included, or whether inclusion was restricted to stage I and II cases only. Comparison of GEP to Breslow thickness, ulceration, and mitotic rate showed that GEP and ulceration were significantly correlated to metastatic risk in a multivariate analysis (HR = 5.0 and 4.2, p = 0.011 and 0.001, respectively). Direct comparison to AJCC stage by multivariate analysis indicates that both factors are significantly correlated to metastasis (GEP HR=7.5, p=0.001; AJCC HR=8.5, p<0.0001). These results indicate that the 28-gene signature is able to predict metastasis independently of AJCC TNM staging and other prognostic factors.

**Table 5: Univariate and multivariate analysis of cutaneous melanoma prognostic factors with inclusion of cases from all AJCC stages.**

| **Variable** | **Univariate** | | | **Multivariate** | | |
|---|---|---|---|---|---|---|
| | HR | 95% CI | *p*-value | HR | 95% CI | *p*-value |
| Breslow | 138.5 | 3.3-5754 | 0.009 | 13.1 | 0.2-972.5 | 0.243 |
| Ulceration | 13.4 | 5.9-30.5 | 4.00E-07 | 4.2 | 1.76-10.1 | 0.001 |
| Mitotic | 2.8 | 0.9-9.3 | 0.089 | 2.0 | 0.6-6.9 | 0.288 |
| GEP test | 20.3 | 7.0-58.9 | 5.13E-07 | 5.0 | 1.4-17.4 | 0.011 |
| AJCC | 21 | 8.6-51.4 | 3.77E-07 | 8.5 | 3.2-22.8 | 2.23E-05 |
| GEP test | 20.3 | 7.0-58.9 | 5.13E-07 | 7.5 | 2.2-25.2 | 0.001 |

An important clinical application of the GEP test is to identify patients who are staged as low risk (clinical stage I and II) for metastasis, but who are at actual high risk for metastasis based upon the genomic signature of their CM. The Cox univariate and multivariate regression analysis was performed for those patients in the 104 sample validation cohort following prediction of metastatic risk (Table 6) who had clinical stage I and II CM only. Again, GEP was compared to the elements comprising AJCC pathologic stage: Breslow thickness, mitotic rate, and ulceration status, and directly to AJCC stage. Under univariate analysis, Breslow's thickness, ulceration status and GEP Class 2 were significant (p<0.0003). However, under multivariate analysis, GEP Class 2, Breslow thickness and ulceration were significant (Hazard ratio = 6.1, 20, and 3.9, respectively; p = 0.02, 0.028, and 0.011, respectively). Univariate analyses for comparison of AJCC stage to GEP Class 2 showed both to be statistically significant, but the GEP Class 2 variable had a greater Hazard ratio than AJCC stage (20.3 vs. 15.2, respectively). Multivariate analyses demonstrated that both factors were independent of each other (p=0.002) but the Hazard Ratio for the GEP Class 2 was again greater than that of AJCC stage (9.6 vs. 5.4). Together, these two result tables (Table 5 and 6) indicate that the 28-gene signature predicts metastasis independently of AJCC TNM staging and other prognostic factors in patients of all stage (I, II, III and IV).

**Table 6: Univariate and multivariate analysis of cutaneous melanoma prognostic factors with inclusion of cases from AJCC stages I and II.**

| **Variable** | **Univariate** | | | **Multivariate** | | |
|---|---|---|---|---|---|---|
| | HR | 95% CI | *p*-value | HR | 95% CI | *p*-value |
| Breslow | 165.6 | 10.7-2559 | 0.0003 | 20 | 1.4-303 | 0.028 |
| Ulceration | 13.1 | 4.8-35.6 | 8.1E-07 | 3.9 | 1.4-10.9 | 0.011 |
| Mitotic | 1.7 | 0.5-5.8 | 0.407 | 1.4 | 0.4-5.2 | 0.595 |
| GEP test | 20.3 | 5.8-70.8 | 2.9E-06 | 6.1 | 1.3-27.6 | 0.020 |
| AJCC | 15.2 | 5.8-39.7 | 6.0E-07 | 5.4 | 1.8-15.7 | 0.002 |
| GEP test | 20.3 | 5.8-70.8 | 2.9E-06 | 9.6 | 2.3-39.5 | 0.002 |

### Example 8: 28-gene signature for cutaneous melanoma predicts metastasis in superficial spreading and nodular growth patterns

Of the four growth patterns described for CM tumors, superficial spreading is the most prevalent, and biologically distinct from nodular melanomas.¹³ Radial basis machine analysis of the superficial spreading tumors within the cohort (n=128) resulted in a highly accurate classification of metastatic and non-metastatic tumors (Figure 5A). K-M MFS analysis shows that cases predicted to be class 1 have a 100% 5-year metastasis free rate, while high risk class 2 cases have only a 5% MFS rate. Additionally, an ROC=1.00 reflects 100% accuracy of the prediction for superficial spreading tumors. The genetic signature is also able to predict metastatic potential for nodular type CM tumors (Figure 5B). K-M MFS rate is 81% for class 1 cases, compared to 7% for class 2 cases (p<0.0001). Accuracy of the predicted model was high, as evidenced by ROC = 0.9323, accuracy of 82%, and sensitivity of 81%. These results indicate that the genetic signature has high accuracy for predicting superficial spreading disease and lower accuracy for predicting nodular disease.

### Example 9: Trimodal and linear methods for predicting metastatic risk according to GEP

While a bimodal (low risk Class 1 vs high risk Class 2) may be the preferred clinical reporting output, additional analysis suggest that the tri-modal or linear approach may be clinically appropriate. Analysis using a trimodal approach demonstrated a stratification outcome related to metastatic risk. Specifically, development of a Class A (low risk), Class B (medium risk) and Class C (high risk) trimodal output shows a graduated increase in metastatic risk (Figure 6). Analysis shows 5-yr MFS for the independent 104 sample validation set is 98% for class A compared to 79% for class B and 30% for class C (*p*<0.0001), with a 64% rate in the overall cohort, reflecting clinically valuable models.

Probability of the metastatic risk prediction is based upon the coefficients of the 28 variables (genes) and reported as a value between 0 and 1. For the two-class analysis, cases with a probability score between 0 and 0.5 are designated low risk, class 1 cases. Conversely, cases with a probability score between 0.5 and 1.0 are classified as class 2, high risk. The linear output of the probability score can also be used directly to assess the risk associated with the genetic signature of particular CM tumor. As shown in Figure 7, 5 year MFS of cases in the 104 sample validation cohort is strongly correlated with the probability of metastasis. Assessment of all cases with a probability of metastasis lower than 0.299 shows that none of those cases has had a documented metastatic event to date, and 5 year MFS is 100%. Four of the 29 cases that fall between the probability scores of 0.3 and 0.499 have evidence of a metastatic event, and 5 year MFS for this group is greater than 92%. Those cases with probability scores between 0.5 and 1.0 have a significant reduction in 5 year MFS. As illustrated by curves F-J in Figure 7, 0.1 increment increases in the probability score lead to corresponding reductions in 5 year MFS from 50% to 45%, 33%, 25%, and 10%. Thus, the utility of the 28 gene predictive signature lies in both the class assignment of metastatic risk and the linear correlation of probability with metastatic outcome.

### Example 10: Prediction of distant metastasis free survival (DMFS) and overall survival (OS) using the GEP prognostic signature.

Initial validation studies to prove the prognostic accuracy of the 28-gene signature and clinically useful training set used metastasis free survival (MFS) as the endpoint for data analysis. MFS reflects the progression of disease to either regional (including the sentinel lymph node) and/or distant sites of melanoma spread. Evaluating the endpoints of DMFS (time to spread beyond the basin of the sentinel lymph node) and OS (time to death) indicates that the GEP signature has accurate prognostic capabilities for predicting both endpoints (Figure 8). Kaplan-Meier analysis was performed for compare MFS, DMFS and OS in two related validation sets, the first consisting of 104 CM cases that includes Stage I-IV melanomas (Figure 8A-C), and a second smaller subset of 78 CM cases that includes only Stage I and Stage II melanomas (Figure 8D-F).

DMFS was determined for the GEP signature, and the results were compared to Breslow thickness, ulceration and mitotic rate, three clinical T-factors commonly assessed to determine AJCC stage for each CM patient (Figure 9). DMFS is grouped by GEP class assignment (Figure 9A), Breslow thickness of greater or less than 0.75mm (Figure 9B), presence or absence of ulceration (Figure 9C), or mitotic rate of greater or less than 1/mm² (Figure 9D). The comparison indicates that the GEP signature has prognostic accuracy better than, or comparable to, the clinical factors currently recommended for the clinical assessment of CM patient risk.

### Example 11: Comparison of prognostic GEP signature to sentinel lymph node biopsy for predicting regional and distant metastasis.

A total of 217 melanoma tumor specimens from patients undergoing sentinel lymph node biopsy (SLNB) had gene expression profiling performed with the 28-gene signature to classify the cases according to risk. A total of 58 patients had a positive SLNB result while 159 patients had a negative SLNB result (Table 7). A total of 37 of 58 (64%) SLNB positive patients developed metastasis and 19 of the 58 (33%) patients died of their disease. Among the SLNB negative patients 70 of 159 (44%) developed metastasis and 46 ultimately died of their disease. The AJCC staging parameters of the SLNB positive and negative patients is summarized in Table 7. The PPV (positive predictive value) of SLNB for metastasis was 64% while the NPV (negative predictive value) of SLNB for metastasis was 56% (Table 8).

**Table 7: Baseline demographics for patients in study cohort who underwent a SLNB procedure.**

| **Clinical Characteristics** | | **SLN positive (n=58)** | **SLN negative (n=159)** |
|---|---|---|---|
| Median Age (range) | | 57 (23-94) | 63 (31-89) |
| Regional/distant mets | | 37 | 70 |
| AJCC Stage | | | |
| | I | n/a | 46 |
| | II | n/a | 112 |
| | III | 58 | 0 |
| | IV | n/a | 1 |
| Breslow Thickness | | | |
| | Median (range) | 4.0 (0.8-16) | 2.3 (0.4-14) |
| | ≤ 1mm | 4 | 27 |
| | > 1mm | 51 | 132 |
| Mitotic Index | | | |
| | < 1/mm2 | 2 | 44 |
| | ≥ 1/mm2 | 48 | 100 |
| Ulceration | | | |
| | absent | 20 | 93 |
| | present | 33 | 58 |

**Table 8: Positive predictive and negative predictive values for SLNB and GEP prognostic tools.**

| **Prognostic Tool** | **PPV,% (95% CI)** | **NPV,% (95% CI)** |
|---|---|---|
| SLNB | 64 (50 - 75) | 56 (48 - 64) |
| GEP | 65 (56 - 72) | 79 (68 - 87) |

Figure 10 shows the KM curves for metastasis free survival (MFS) and overall survival (OS) for the SLNB negative and the SLNB positive patients. The respective*p-*values for the difference between the curves for the SLNB positive and SLNB negative patients was <0.0001 and <0.006.

Among the 217 melanoma patients, 141 had a high risk Class 2 GEP result while 76 had a low risk Class 1 result. Ninety-one of the 141 Class (65%) 2 patients developed metastatic disease while 55 died of disease. Sixteen of 76 (21%) Class 1 patients developed metastatic disease and 10 of 76 (13%) died of disease. The PPV of the GEP test for metastasis was 65% and the NPV was 79%. Figure 11 shows the KM curve for MFS and OS for the GEP classification of patients. The respective *p*-values for the difference between the curves for the GEP Class 1 and Class 2 patients was<0.0001 for both analyses. The OS at 5 years for all SLNB positive patients was 62% compared to 54% for cases with a Class 2 GEP score. The OS at 5 years for all SLNB negative cases was 70%, compared to 89% for all cases with a Class 1 GEP score.

There was a statistically significant difference in univariate and multivariate analysis of a SLNB positive result (compared to SLNB negative) and the GEP Class 2 score (compared to GEP Class 1) when comparing the metastatic and non-metastatic groups with Cox regression (Table 9). The respective *p*-values for the multivariate analysis were <0.008 and <3.1x10⁻⁵. Likewise both SLNB result and the GEP score were statistically significant when comparing cases with death of disease versus all others with Cox regression analysis in univariate analysis. The respective *p*-values being <0.007 and <1.9x10⁻⁶. Conversely, in multivariate analysis only the GEP score was statistically significant with a *p*-value of <5.2x10⁻⁶.

**Table 9: Positive predictive and negative predictive values for SLNB and GEP prognostic tools.**

| | | **Univariate** | | | **Multivariate** | | |
|---|---|---|---|---|---|---|---|
| | **Variable** | **HR** | **95% CI** | ***p*-value** | **HR** | **95% CI** | ***p*-value** |
| **MFS** | GEP Class 2 | 5.5 | 3.2-9.4 | 4.0E-07 | 4.9 | 2.9-8.6 | 3.1E-05 |
| | SLN+ | 2.4 | 1.6-3.6 | 2.6E-05 | 1.7 | 1.2-2.6 | 0.008 |
| **OS** | GEP Class 2 | 5.4 | 2.7-10.8 | 1.9E-06 | 5.1 | 2.5-10.2 | 5.2E-06 |
| | SLN+ | 2.1 | 1.2-3.6 | 0.007 | 1.6 | 0.9-2.8 | 0.092 |

Figure 12 illustrates that using GEP classification in combination with SLNB status further improves prognostication. The overall 5-year MFS and 5-year OS for Class 1 patients is 79% and 89%, respectively, and for SLNB negative patients is 55% and 70%, respectively. The 31% of patients with a GEP Class 1 and a negative SLNB result had an overall 5-year MFS and 5-year OS of 83% and 91%, respectively. The overall 5-year MFS and 5 year OS for GEP Class 2 patients is 34% and 54% respectively and for SLNB positive patients is 37% and 62%. The 23% of patients with a GEP Class 2 and a positive SLNB had an overall 5-year MFS and 5 year OS of 33% and 57%. For the 42% of patients with a GEP Class 2 but negative SLNB result, the 5-year MFS and 5-year OS were 35% and 54%, respectively, similar to those patients who were Class 2 with a positive SLNB result. Only a small cohort of 9 patients (representing 4%) with a Class 1 GEP score, but positive SLNB result, was identified. The overall 5-year MFS and 5-year OS for this group was 53% and 78%, respectively. The respective *p*-values for the difference between the four combined GEP and SLNB groups was<0.0001 for both MFS and OS analyses.

Prediction of MFS and DMFS when combining GEP Class with SLNB outcome is shown in Figure 13. Cases with Class 1/SLN- results continue to exhibit the lowest risk of distant metastasis, with 86% 5-year DMFS rates. Conversely, Class 2/SLN+ cases have the highest risk of developing distant disease, and have a 42% DMFS rate. As shown in Figure 14, adding the GEP signature to AJCC staging provided a surgical yield of 35% (49/141) compared to 27% (58/217) with AJCC staging alone. A prognostic advantage was also observed for the GEP, as SLNB procedure (C) identified 37/107 cases (sensitivity = 35%) with a documented metastatic event, while adding GEP to SLNB (D) identified 91/107 cases (sensitivity = 85%) with a documented metastatic event.

Each embodiment herein described may be combined with any other embodiment or embodiments unless clearly indicated to the contrary. In particular, any feature or embodiment indicated as being preferred or advantageous may be combined with any other feature or features or embodiment or embodiments indicated as being preferred or advantageous, unless clearly indicated to the contrary.

### References

1. Siegel R, Naishadham D, Jemal A. Cancer statistics, 2012. CA Cancer J Clin 2012;62:10-29.
2. Balch CM, Soong SJ, Gershenwald JE, et al. Prognostic factors analysis of 17,600 melanoma patients: validation of the American Joint Committee on Cancer melanoma staging system. J Clin Oncol 2001;19:3622-34.
3. Balch CM, Gershenwald JE, Soong SJ, et al. Final version of 2009 AJCC melanoma staging and classification. J Clin Oncol 2009;27:6199-206.
4. Balch CM, Buzaid AC, Soong SJ, et al. Final version of the American Joint Committee on Cancer staging system for cutaneous melanoma. J Clin Oncol 2001;19:3635-48.
5. Edge SB, Compton CC. The American Joint Committee on Cancer: the 7th edition of the AJCC cancer staging manual and the future of TNM. Ann Surg Oncol 2010;17:1471-4.
6. Takeuchi H, Morton DL, Kuo C, et al. Prognostic significance of molecular upstaging of paraffin-embedded sentinel lymph nodes in melanoma patients. J Clin Oncol 2004;22:2671-80.
7. Zettersten E, Shaikh L, Ramirez R, Kashani-Sabet M. Prognostic factors in primary cutaneous melanoma. Surg Clin North Am 2003;83:61-75.
8. Balch CM, Gershenwald JE, Soong SJ, et al. Multivariate analysis of prognostic factors among 2,313 patients with stage III melanoma: comparison of nodal micrometastases versus macrometastases. J Clin Oncol 2010;28:2452-9.
9. Yee VS, Thompson JF, McKinnon JG, et al. Outcome in 846 cutaneous melanoma patients from a single center after a negative sentinel node biopsy. Ann Surg Oncol 2005;12:429-39.
10. Karim RZ, Scolyer RA, Li W, et al. False negative sentinel lymph node biopsies in melanoma may result from deficiencies in nuclear medicine, surgery, or pathology. Ann Surg 2008;247:1003-10.
11. Nicholl MB, Elashoff D, Takeuchi H, Morton DL, Hoon DS. Molecular upstaging based on paraffin-embedded sentinel lymph nodes: ten-year follow-up confirms prognostic utility in melanoma patients. Ann Surg 2011;253:116-22.
12. Coit DG. Melanoma Version 2.2013: National Comprehensive Cancer Network; 2013 2/2013.
13. Morton DL. Overview and update of the phase III Multicenter Selective Lymphadenectomy Trials (MSLT-I and MSLT-II) in melanoma. Clin Exp Metastasis 2012;29:699-706.
14. Onken MD, Worley LA, Ehlers JP, Harbour JW. Gene expression profiling in uveal melanoma reveals two molecular classes and predicts metastatic death. Cancer Res 2004;64:7205-9.
15. Francis P, Namlos HM, Muller C, et al. Diagnostic and prognostic gene expression signatures in 177 soft tissue sarcomas: hypoxia-induced transcription profile signifies metastatic potential. BMC Genomics 2007;8:73.
16. Paik S, Shak S, Tang G, et al. A multigene assay to predict recurrence of tamoxifentreated, node-negative breast cancer. N Engl J Med 2004;351:2817-26.
17. Colman H, Zhang L, Sulman EP, et al. A multigene predictor of outcome in glioblastoma. Neuro Oncol 2010;12:49-57.
18. Gordon GJ, Jensen RV, Hsiao LL, et al. Using gene expression ratios to predict outcome among patients with mesothelioma. J Natl Cancer Inst 2003;95:598-605.
19. Sato T. Locoregional management of hepatic metastasis from primary uveal melanoma. Semin Oncol 2010;37:127-38.
20. Onken MD, Worley LA, Tuscan MD, Harbour JW. An accurate, clinically feasible multi-gene expression assay for predicting metastasis in uveal melanoma. J Mol Diagn 2010;12:461-8.
21. Onken MD, Worley LA, Char DH, et al. Collaborative Ocular Oncology Group Report Number 1: Prospective Validation of a Multi-Gene Prognostic Assay in Uveal Melanoma. Ophthalmology 2012.
22. Jaeger J, Koczan D, Thiesen HJ, et al. Gene expression signatures for tumor progression, tumor subtype, and tumor thickness in laser-microdissected melanoma tissues. Clin Cancer Res 2007;13:806-15.
23. Bittner M, Meltzer P, Chen Y, et al. Molecular classification of cutaneous malignant melanoma by gene expression profiling. Nature 2000;406:536-40.
24. Haqq C, Nosrati M, Sudilovsky D, et al. The gene expression signatures of melanoma progression. Proc Natl Acad Sci U S A 2005;102:6092-7.
25. Mauerer A, Roesch A, Hafner C, et al. Identification of new genes associated with melanoma. Exp Dermatol 2011;20:502-7.
26. Scatolini M, Grand MM, Grosso E, et al. Altered molecular pathways in melanocytic lesions. Int J Cancer 2010;126:1869-81.
27. Smith AP, Hoek K, Becker D. Whole-genome expression profiling of the melanoma progression pathway reveals marked molecular differences between nevi/melanoma in situ and advanced-stage melanomas. Cancer Biol Ther 2005;4:1018-29.
28. Weeraratna AT, Becker D, Carr KM, et al. Generation and analysis of melanoma SAGE libraries: SAGE advice on the melanoma transcriptome. Oncogene 2004;23:2264-74.
29. Winnepenninckx V, Lazar V, Michiels S, et al. Gene expression profiling of primary cutaneous melanoma and clinical outcome. J Natl Cancer Inst 2006;98:472-82.

## Claims

1. A method for predicting risk of metastasis, overall survival, or both, in a patient with a primary cutaneous melanoma tumor, the method comprising:
(a) measuring gene expression levels of at least eight genes selected from: BAP1_varA, BAP1_varB, MGP, SPP1, CXCL14, CLCA2, S100A8, BTG1, SAP130, ARG1, KRT6B, GJA, ID2, EIF1B, S100A9, CRABP2, KRT14, ROBO1, RBM23, TACSTD2, DSC1, SPRR1B, TRIM29, AQP3, TYRP1, PPL, LTA4H, and CST6, in a sample of the primary cutaneous melanoma tumor,
wherein the at least eight genes are:
SPP1, MGP, KRT6B, PPL, RBM23, AQP3, CXCL14, and GJA1;
wherein measuring gene expression levels of the at least eight genes comprises measurement of a level of fluorescence by a sequence detection system following RT-PCR of the at least eight genes;
(b) determining a patient gene-expression profile signature comprising the gene expression levels of the at least eight genes;
(c) comparing the patient gene-expression profile signature to a gene-expression profile of a predictive training set; and
(d) providing an indication as to a risk of metastasis, overall survival or both for the primary cutaneous melanoma tumor when the patient gene expression profile indicates that the expression levels of at the least eight genes are altered in a predictive manner as compared to the gene expression profile of the predictive training set.

2. The method of claim 1, wherein the risk of metastasis for the primary cutaneous melanoma tumor is classified from a low risk of metastasis to a high risk of metastasis.

3. The method of claim 2, wherein class 1 indicates a low risk of metastasis, class 2 indicates a high risk of metastasis, class A indicates a low risk of metastasis, class B indicates an intermediate risk of metastasis, and class C indicates a high risk of metastasis.

4. The method of any one of claims 1 to 3, further comprising determining that the primary cutaneous melanoma tumor has an increased risk of metastasis or decreased overall survival by combining with at least one of TNM (Tumor-Node-Metastasis) status, clinical staging set by American Joint Committee on Cancer (AJCC) to stage the primary cutaneous melanoma tumor, and sentinel lymph node biopsy status.

5. The method of any one of claims 1 to 4, wherein a sentinel lymph node biopsy was performed in the patient from which the primary cutaneous melanoma tumor was separately obtained.

6. The method of claim 5, wherein the sentinel lymph node biopsy was negative.

7. The method of any one of claims 1 to 6, wherein the primary cutaneous melanoma tumor is taken from a formalin-fixed, paraffin embedded wide local excision sample.

8. The method of any one of claims 1 to 7, wherein the primary cutaneous melanoma tumor is taken from formalin-fixed, paraffin embedded biopsy sample selected from a punch biopsy, a shave biopsy, and another biopsy method.

## Patentansprüche

1. Verfahren zur Vorhersage des Risikos von Metastasen, des Gesamtüberlebens oder von beidem bei einem Patienten mit einem primären Hautmelanomtumor, wobei das Verfahren Folgendes umfasst:
(a) Messen von Genexpressionsniveaus von mindestens acht Genen, die aus Folgenden ausgewählt sind: BAP1_varA, BAP1_varB, MGP, SPP1, CXCL14, CLCA2, S100A8, BTG1, SAP130, ARG1, KRT6B, GJA, ID2, EIF1B, S100A9, CRABP2, KRT14, ROBO1, RBM23, TACSTD2, DSC1, SPRR1B, TRIM29, AQP3, TYRP1, PPL, LTA4H und CST6, in einer Probe des primären Hautmelanomtumors,
wobei die mindestens acht Gene Folgende sind:
SPP1, MGP, KRT6B, PPL, RBM23, AQP3, CXCL14, und GJA1;
wobei das Messen von Genexpressionsniveaus der mindestens acht Gene Messen eines Fluoreszenzniveaus durch ein Sequenznachweissystem nach RT-PCR der mindestens acht Gene umfasst;
(b) Bestimmen einer Patientengenexpressionsprofilsignatur, welche die Genexpressionsniveaus der mindestens acht Gene umfasst;
(c) Vergleichen der Patientengenexpressionsprofilsignatur mit einem Genexpressionsprofil eines vorhersagenden Trainingssatzes; und
(d) Bereitstellen einer Angabe eines Risikos von Metastasen, des Gesamtüberlebens oder von beiden für den primären Hautmelanomtumor, wenn das Patientengenexpressionsprofil angibt, dass die Expressionsniveaus von mindestens acht Genen im Vergleich zu dem Genexpressionsprofil des vorhersagenden Trainingssatzes auf vorhersagende Weise verändert sind.

2. Verfahren nach Anspruch 1, wobei das Risiko von Metastasen für den primären Hautmelanomtumor von einem geringen Risiko von Metastasen bis zu einem hohen Risiko von Metastasen klassifiziert wird.

3. Verfahren nach Anspruch 2, wobei Klasse 1 ein geringes Risiko von Metastasen angibt, Klasse 2 ein hohes Risiko von Metastasen angibt, Klasse A ein geringes Risiko von Metastasen angibt, Klasse B ein mittleres Risiko von Metatasten angibt und Klasse C ein hohes Risiko von Metastasen angibt.

4. Verfahren nach einem der Ansprüche 1 bis 3, ferner umfassend Bestimmen, dass der primäre Hautmelanomtumor ein erhöhtes Risiko von Metastasen oder ein verringertes Gesamtüberleben aufweist, durch Kombinieren mit mindestens einem von einem TNM-Status (Tumor-Knoten-Metastasen), eines klinischen Staging, das vom American Joint Committee on Cancer (AJCC) festgelegt wurde, um den primären Hautmelanomtumor durch Staging einzuschätzen, und einem Sentinel-Lymphknoten-Biopsie-Status.

5. Verfahren nach einem der Ansprüche 1 bis 4, wobei eine Sentinel-Lymphknotenbiopsie bei dem Patienten durchgeführt wurde, von dem der primäre Hautmelanomtumor separat erhalten wurde.

6. Verfahren nach Anspruch 5, wobei die Sentinel-Lymphknotenbiopsie negativ war.

7. Verfahren nach einem der Ansprüche 1 bis 6, wobei der primäre Hautmelanomtumor einer formalinfixierten, in Paraffin eingebetteten breiten lokalen Exzisionsprobe entnommen wird.

8. Verfahren nach einem der Ansprüche 1 bis 7, wobei der primäre Hautmelanomtumor einer formalinfixierten, in Paraffin eingebetteten Biopsieprobe entnommen wird, die aus einer Stanzbiopsie, einer Rasurbiopsie und einem anderen Biopsieverfahren ausgewählt ist.

## Revendications

1. Méthode de prédiction du risque de métastase, de survie globale, ou des deux, chez un patient ayant une tumeur primaire de mélanome cutané, la méthode comprenant les étapes consistant à :
(a) mesurer les niveaux d'expression génique d'au moins huit gènes choisis parmi : BAP1_varA, BAP1_varB, MGP, SPP1, CXCL14, CLCA2, S100A8, BTG1, SAP130, ARG1, KRT6B, GJA, ID2, EIF1B, S100A9, CRABP2, KRT14, ROBO1, RBM23, TACSTD2, DSC1, SPRR1B, TRIM29, AQP3, TYRP1, PPL, LTA4H et CST6, dans un échantillon de la tumeur primaire de mélanome cutané,
où les au moins huit gènes sont :
SPP1, MGP, KRT6B, PPL, RBM23, AQP3, CXCL14 et GJA1 ;
où la mesure des niveaux d'expression génique des au moins huit gènes comprend la mesure d'un niveau de fluorescence par un système de détection de séquence suivant une amplification en chaîne par polymérase en temps réel (RT-PCR) des au moins huit gènes ;
(b) déterminer une signature de profil d'expression génique du patient comprenant les niveaux d'expression génique des au moins huit gènes ;
(c) comparer la signature de profil d'expression génique du patient à un profil d'expression génique d'un ensemble d'apprentissage prédictif ; et
(d) fournir une indication d'un risque de métastase, de survie globale ou des deux pour la tumeur primaire de mélanome cutané lorsque le profil d'expression génique du patient indique que les niveaux d'expression des au moins huit gènes sont modifiés d'une manière prédictive par rapport au profil d'expression génique de l'ensemble d'apprentissage prédictif.

2. Méthode selon la revendication 1, dans laquelle le risque de métastase pour la tumeur primaire de mélanome cutané est classé d'un risque faible de métastase à un risque élevé de métastase.

3. Méthode selon la revendication 2, dans laquelle la classe 1 indique un risque faible de métastase, la classe 2 indique un risque élevé de métastase, la classe A indique un risque faible de métastase, la classe B indique un risque intermédiaire de métastase et la classe C indique un risque élevé de métastase.

4. Méthode selon l'une quelconque des revendications 1 à 3, comprenant en outre l'étape consistant à déterminer que la tumeur primaire de mélanome cutané a un risque accru de métastase ou une survie globale réduite en se combinant avec au moins l'un du statut TNM (tumeur-ganglion-métastase), de la classification clinique établie par l'American Joint Committee on Cancer (AJCC) pour classer la tumeur primaire de mélanome cutané et du statut de la biopsie du ganglion lymphatique sentinelle.

5. Méthode selon l'une quelconque des revendications 1 à 4, dans laquelle une biopsie du ganglion lymphatique sentinelle a été effectué chez le patient d'où la tumeur de mélanome primaire cutané a été obtenue séparément.

6. Méthode selon la revendication 5, dans laquelle la biopsie du ganglion lymphatique sentinelle était négative.

7. Méthode selon l'une quelconque des revendications 1 à 6, dans laquelle la tumeur primaire de mélanome cutané est prélevée à partir d'un échantillon d'excision locale large fixé au formol et enrobé dans la paraffine.

8. Méthode selon l'une quelconque des revendications 1 à 7, dans laquelle la tumeur primaire de mélanome cutané est prélevée à partir d'un échantillon de biopsie fixé au formol et enrobé dans la paraffine, sélectionné à partir d'une biopsie à l'emporte-pièce, d'une biopsie par rasage et d'une autre méthode de biopsie.
